# EUROPEAN PATENT SPECIFICATION

(11) **EP 0 565 634 B1**
(45) Date of publication and mention of the grant of the patent: **17.03.1999**
(21) Application number: 92904382.6
(22) Date of filing: 13.12.1991
(51) Int. Cl.: A61K 31/415, A61K 31/54, A61K 45/06, A61K 38/55, A61K 31/44, A61K 31/55

(54) **ANGIOTENSIN II RECEPTOR BLOCKING COMPOSITIONS**
ANGIOTENSIN-II-REZEPTOR BLOCKIERENDE ZUSAMMENSETZUNGEN
COMPOSITIONS DE BLOCAGE DE RECEPTEUR DE L'ANGIOTENSINE II

(30) Priority: 14.12.1990 US 628807
(43) Date of publication of application: 20.10.1993
(73) Proprietor: SMITHKLINE BEECHAM CORPORATION, Philadelphia, PA 19102 (US)
(72) Inventor: WEINSTOCK, Joseph SmithKline Beecham Pharmac. R&D, King of Prussia, PA 19406 (US)
(74) Representative: Thompson, Clive Beresford
(86) International application number: US9109362
(87) International publication number: WO9210097

(56) References cited:
- EP-A- 0 324 377
- EP-A- 0 403 159
- PHARMACOL. COMMUN., vol.2, no.4, 1993 pages 331 - 337 D.P. BROOKS 'Blood pressure lowering activity of enalapril and the non-peptide angiotensin-II receptor antagonist, SK&F 108566, in furosemide-treated conscious cynomolgus monkeys.'
- STN INTERNATIONAL, KARLSRUHE. FILE 'PHAR'. AN=10943
- CHEMICAL ABSTRACTS, Volume 115, No. 17, issued 28 October 1991, (Columbus, Ohio, USA), KEENAN et al., "Preparation of 4-(Tetrazol-5-Yl)Alkenylimidazoles as Angiotensin II Antagonists", (SmithKline Beecham Corporation), Abstract No. 183309t; & EP,A,425 211.
- CHEMICAL ABSTRACTS, Volume 115, No. 3, issued 22 July 1991, (Columbus, Ohio, USA), FINKELSTEIN et al., "Preparation of Imidazolyl-4-Alkenoic Acids as Angiotensin II Antagonists", (SmithKline Beecham Corporation), Abstract No. 29327r; & EP,A,403 158.
- CHEMICAL ABSTRACTS, Volume 114, No. 21, issued 27 May 1991, (Columbus, Ohio, USA), FINKELSTEIN et al., "Preparation of Imidazolylalkenoic Acids as Anti-Hypertensives", (SmithKline Beecham Corporation), Abstract No. 207258h; & EP,A,403 159.

## Description

### FIELD OF INVENTION

This invention relates to pharmaceutical compositions comprising angiotensin II receptor antagonists, combined with other agents, particularly diuretics, calcium channel blockers, β-adrenoceptor blockers, renin inhibitors, or angiotensin converting enzyme inhibitors. The invention also relates to the use of said compositions in the manufacture of a medicament for the treatment of hypertension.

### BACKGROUND OF THE INVENTION

The class of peptide pressor hormone known as angiotensin is responsible for a vasopressor action that is implicated in the etiology of hypertension in man. Inappropriate activity of the renin-angiotensin systems appears to be a key element in essential hypertension, congestive heart failure and in some forms of renal disease. In addition to a direct action on arteries and arterioles, angiotensin II (AII), being one of the most potent endogenous vasoconstrictors known, exerts stimulation on the release of aldosterone from the adrenal cortex. Therefore, the renin-angiotensin system, by virtue of its participation in the control of renal sodium handling, plays an important role in cardiovascular hemeostasis.

Interruption of the renin-angiotensin system with converting enzyme inhibitors, such as captopril, has proved to be clinically useful in the treatment of hypertension and congestive heart failure (Abrams, W.B., et al., (1984), Federation Proc., 43, 1314). The most direct approach towards inhibition of the renin-angiotensin system would block the action of AII at the receptor. Compelling evidence suggests that AII also contributes to renal vasoconstriction and sodium retention that is characteristic of a number of disorders such as heart failure, cirrhosis and complications of pregnancy (Hollenberg, N.K., (1984), J. Cardiovasc. Pharmacol., 6, S176). In addition, recent animal studies suggest that inhibition of the renin-angiotensin system may be beneficial in halting or slowing the progression of chronic renal failure (Anderson, S., et al., (1985), J. Clin. Invest., 76, 612). Also, a recent patent application (South African Patent Application No. 87/01, 653)claims that AII antagonists are useful as agents for reducing and controlling elevated intraocular pressure, especially glaucoma, in mammals.

The compounds described herein inhibit, block and antagonize the action of the hormone AII, and are therefore useful in regulating and moderating angiotensin induced hypertension, congestive heart failure, renal failure and other disorders attributed to the actions of AII. When the AII receptor blocking compounds are administered to mammals, the elevated blood pressure due to AII is reduced and other manifestations based on AII intercession are minimized and controlled. These compounds are also expected to exhibit diuretic activity.

One of the drawbacks of current antihypertensive therapy is that cardiovascular morbidity and mortality is higher in treated hypertensive patients than in normotensive subjects of the same age, sex, and from the same population [Isles, et al., J. Hypertension, 4, 141 (1986) and Samuelsson, O., Acta Med. Scand., Suppl. 702, 1 (1985)]. A possible explanation for this result is that arterial pressure in treated hypertensive patients is significantly higher than in matched normotensive subjects. Thus, it would appear that a therapeutic goal for the treatment of hypertension would be to obtain normotensive blood pressure levels in treated hypertensive patients [Hansson, L., Br. J. Clin. Pharmacol., 23, 15S (1987)]. To achieve this goal, the instant invention involves the administration of an angiotensin II receptor blocking compound in combination with a second agent, such as a diuretic, a calcium channel blocker, a β-adrenoceptor blocker, a renin inhibitor, or an angiotensin converting enzyme inhibitor.

According to the present invention there are provided pharmaceutical compositions comprising a pharmaceutically acceptable carrier in association with a diuretic and an angiotensin II blocking agent of formula (I), hereinbelow.

Substituted imidazoles of the formula (I) are disclosed in U.S. Application Serial No. 07/746,262, filed August 14, 1991: 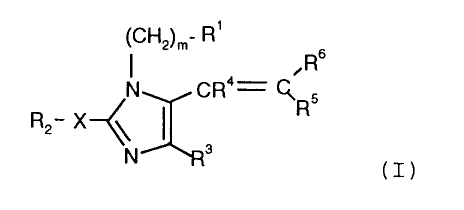 in which:
R¹ is adamantyl, phenyl, biphenyl, or naphthyl, with each aryl group being unsubstituted or substituted by one to three substituents selected from Cl, Br, F, I, C₁ - C₆ alkyl, nitro, A-CO₂R⁷, tetrazol-5-yl, C₁-C₆alkoxy, hydroxy, SC₁-C₆alkyl, SO₂NHR⁷, NHSO₂R⁷, SO₃H, CONR⁷R⁷, CN, SO₂C₁-C₆alkyl, NHSO₂R⁷, PO(OR⁷)₂, NR⁷R⁷, NR⁷COH, NR⁷COC₁-C₆alkyl, NR⁷CON(R⁷)₂, NR⁷COW, W, or SO₂W;
m is 0-4;
R² is C₂-C₁₀alkyl, C₃-C₁₀alkenyl, C₃-C₁₀alkynyl, C₁-C₆cycloalkyl, or (CH₂)₀₋₈phenyl unsubstituted or substituted by one to three substituents selected from C₁-C₆alkyl, nitro, Cl, Br, F, I, hydroxy, C₁-C₆alkoxy, NR⁷R⁷, CO₂R⁷, CN, CONR⁷R⁷, W, tetrazol-5-yl, NR⁷COC₁₋C₆alkyl, NR⁷COW, SC₁-C₆alkyl, SO₂W, or SO₂C₁-C₆alkyl;
X is a single bond, S, NR⁷, or O;
R³ is hydrogen, Cl, Br, F, I, CHO, hydroxymethyl, COOR⁷, CONR⁷R⁷,NO₂, W, CN, NR⁷R⁷, or phenyl;
R⁴ and R⁵ are independently hydrogen, C₁-C₆alkyl, thienyl-Y-, furyl-Y-, pyrazolyl-Y-, imidazolyl-Y-pyrrolyl-Y-, triazolyl-Y-, oxazolyl-Y-, isoxazolyl-Y-, thiazolyl-Y-, pyridyl-Y-, or tetrazolyl-Y-, except that R⁴ and R⁵ are not both selected from hydrogen and C₁-C₆alkyl and each heterocyclic ring is unbsubstituted or substituted by C₁-C₆alkyl, C₁-C₆alkoxy, Cl, Br, F, I, NR⁷R⁷, CO₂R⁷, SO₂NHR⁷, SO₃H, CONR²R⁷, OH, NO₂, W, SO₂W, SC₁-C₆alkyl, SO₂-C₁-C₆alkyl, NR⁷COH, NR⁷COW, or NR⁷COC₁₋C₆alkyl;
Y is a single bond, O, S, or C₁-C₆alkyl which is straight or branched or optionally substituted by phenyl or benzyl, wherein each of the aryl groups is unsubstituted or substituted by halo, NO₂, CF₃, C₁-C₆alkyl, C₁-C₆alkoxy, CN, or CO₂R⁷;
R⁶ is -Z-COOR⁸ or -Z-CONR⁷R⁷;
Z is a single bond, vinyl, -CH₂-O-CH₂-, methylene optionally substituted by C₁-C₆alkyl, one or two benzyl groups, thienylmethyl, or furylmethyl, or -C(O)NHCR⁹-, wherein R⁹ is H, C₁-C₆alkyl, phenyl, benzyl, thienylmethyl, or furylmethyl;
W is CₙF₂ₙ₊₁, wherein n is 1-3;
A is -(CH₂)ₘ-, -CH=CH-, -O(CH₂)ₙ-, or -S(CH₂)ₙ-;
each R⁷ independently is hydrogen, C₁-C₆alkyl, or (CH₂)ₘphenyl, wherein m is 0-4; and
R⁸ is hydrogen, C₁-C₆alkyl, or 2-di(C₁-C₆alkyl)amino-2-oxoethyl; or a pharmaceutically acceptable salt thereof.

Preferred compounds included within the scope of formula (I) are:
(E)-3-[2-n-butyl-1-{(4-carboxyphenyl)methyl}-1H-imidazol-5-yl]-2-(2-thienyl)methyl-2-propenoic acid,
(E)-3-[2-n-butyl-1-{(4-carboxynapth-1-yl)methyl}- 1H-imidazol-5-yl]-2-(2-thienyl)methyl-2-propenoic acid,
(E)-3-[2-n-butyl-1-{(2-chloro-4-carboxyphenyl)methyl}-1H-imidazol-5-yl]-2-(2-thienyl)methyl-2-propenoic acid, and
(E)-3-[2-n-butyl-1-{(4-carboxy-2,3-dichlorophenyl)methyl}-1H-imidazol-5-yl]-2-(2-thienyl)methyl-2-propenoic acid; or a pharmaceutically acceptable salt thereof.

Particularly preferred are (E)-3-[2-n-butyl-1-{(4-carboxyphenyl)methyl}-1H-imidazol-5-yl]-2-(2-thienyl)methyl-2-propenoic acid and (E)-3-[2-n-butyl-1-{(4-carboxynaphth-1-yl)methyl)-1H-imidazol-5-yl]-2-(2-thienyl)methyl-2-propenoic acid, or a pharmaceutically acceptable salt thereof.

The most preferred compound of this invention is (E)-3-[2-n-butyl-1-{(4-carboxyphenyl)methyl}-1H- imidazolyl-5-yl]-2-(2-thienyl)methyl-2-propenoic acid methanesulfonate.

Compounds of formula (I) are prepared following the methods described in European Patent Publication Number EP 0 403 159, published on December 19, 1990.

The above descriptions on pages 3-6 of classes of AII receptor antagonists for use in the present invention were taken from the noted patent application and publication. Reference should be made to such patent application and publication for their full disclosure, the entire disclosure of each of which is incorporated herein by reference.

Also included within the scope of this invention are pharmaceutical compositions comprising a pharmaceutically acceptable carrier in association with a compound of formula (I) and a second therapeutic agent, such as a diuretic, a calcium channel blocker, a β-adrenoceptor blocker, a renin inhibitor, or an angiotensin converting enzyme inhibitor.

This invention also relates to the use of a compound of formula (I) stepwise or in physical combination with a diuretic, a calcium channel blocker, a β-adrenoceptor blocker, a renin inhibitor, or an angiotensin converting enzyme inhibitor in the manufacture of a medicament for the treatment of hypertension.

Most advantageously the compositions of this invention in dosage unit form are comprised of (E)-3-[2-n-butyl-1-{(4-carboxyphenyl)methyl}-1H-imidazol-5-yl]-2-(2-thienyl)methyl-2-propenoic acid, or a pharmaceutically acceptable salt thereof, and a diuretic, a calcium channel blocker, a β-adrenoceptor blocker, a renin inhibitor, or an angiotensin converting enzyme inhibitor. Either can alternatively be used in the form of a non toxic salt.

Pharmaceutically acceptable acid addition salts of compounds of Formula (I) are formed with appropriate organic or inorganic acids by methods known in the art. For example, the base is reacted with a suitable inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Representative examples of suitable acids are maleic, fumaric, benzoic, ascorbic, pamoic, succinic, bismethylenesalicylic, methanesulfonic, ethanedisulfonic, acetic, propionic, tartaric, salicylic, citric, gluconic, aspartic, stearic, palmitic, itaconic, glycolic, p-aminobenzoic, flutamic, benzenesulfonic, hydrochloric, hydrobromic, sulfuric, cyclohexysulfamic, phosphoric and nitric acids.

Pharmaceutically acceptable base addition salts of compounds of Formula (I) wherein a carboxy group is present and prepared by known methods from organic and inorganic bases, including nontoxic alkali metal and alkaline earth bases, for example, calcium, lithium, sodium, and potassium hydroxide; ammonium hydroxide, and nontoxic organic bases, such as triethylamine, butylamine, piperazine, meglumine, choline, diethanolamine, and trimethamine.

Angiotensin II antagonist activity of the compounds of Formula (I) is assessed by in vitro and in vivo methods. In vitro antagonist activity is determined by the ability of the compounds to compete with ¹²⁵I-angiotensin II for binding to vascular angiotensin II receptors and by their ability to antagonize the contractile response to angiotensin II in the isolated rabbit aorta. In vivo activity is evaluated by the efficacy of the compounds to inhibit the pressor response to exogenous angiotensin II in conscious rats and to lower blood pressure in a rat model of renin dependent hypertension.

### Binding

The radioligand binding assay is a modification of a method previously described in detail (Gunther et al., Circ. Res. 47:278, 1980). A particular fraction from rat mesenteric arteries is incubated in Tris buffer with 80 pM of ¹²⁵I-angiotensin II with or without angiotensin II antagonists for 1 hour at 25°C. The incubation is terminated by rapid filtration and receptor bound ¹²⁵I-angiotensin II trapped on the filter is quantitated with a gamma counter. The potency of angiotensin II antagonists is expressed as the IC₅₀ which is the concentration of antagonist needed to displace 50% of the total specifically bound angiotensin II. The IC₅₀ of (E)-3-[2-n-butyl-1-{(4-carboxyphenyl)methyl}-1H-imidazol-5-yl]-2-(2-thienyl)methyl-2-propenoic acid is about 1.0 nM.

### Aorta

The ability of the compounds to antagonize angiotensin II induced vasoconstriction is examined in the rabbit aorta. Ring segments are cut from the rabbit thoracic aorta and suspended in organ bathes containing physiological salt solution. The ring segments are mounted over metal supports and attached to force displacement transducers which are connected to a recorder. Cumulative concentration response curves to angiotensin II are performed in the absence of antagonist or following a 30-minute incubation with antagonist. Antagonist disassociation constants (K_{B}) are calculated by the dose ratio method using the mean effective concentrations. The K_{B} of (E)-3-[2-n-butyl-1-{(4-carboxyphenyl)methyl}-1H-imidazol-5-yl]-2-(2-thienyl)methyl-2-propenoic acid is about 0.20 nM.

### Inhibition of pressor response to angiotensin II in conscious rats

Rats are prepared with indwelling femoral arterial and venous catheters and a stomach tube (Gellai et al., Kidney Int. 15:419, 1979). Two to three days following surgery the rats are placed in a restrainer and blood pressure is continuously monitored from the arterial catheter with a pressure transducer and recorded on a polygraph. The change in mean arterial pressure in response to intravenous injections of 250 mg/kg angiotensin II is compared at various time points prior to and following the administration of the compounds intravenously or orally at doses of 0.1 to 300 mg/kg. The dose of compound needed to produce 50% inhibition of the control response to angiotensin II (IC₅₀) is used to estimate the potency of the compounds. The IC₅₀ of (E)-3-[2-n-butyl-1-{(4-carboxyphenyl)methyl}-1H-imidazol-5-yl]-2-(2-thienyl)methyl-2-propenoic acid is about 0.1 mg/kg i.v. and about 5.5 mg/kg orally.

### Antihypertensive activity

The antihypertensive activity of the compounds is measured by their ability to reduce mean arterial pressure in conscious rats made renin-dependent hypertensive by ligation of the left renal artery (Cangiano et al;., J. Pharmacol. Exp. Ther. 208:310, 1979). Renal artery ligated rats are prepared with indwelling catheters as described above. Seven to eight days following renal artery ligation, the time at which plasma renin levels are highest, the conscious rats are placed in restrainers and mean arterial pressure is continuously recorded prior to and following the administration of the compounds intravenously or orally. The dose of compound needed to reduce mean arterial pressure by 30 mmHg (IC₃₀) is used as an estimate of potency. The IC₃₀ of (E)-3-[2-n-butyl-1-{(4-carboxyphenyl)methyl}-1H-imidazol-5-yl]-2-(2-thienyl)methyl-2-propenoic acid is about 10.0 mg/kg orally.

The antihypertensive activity of the claimed pharmaceutical composition is determined using the spontaneously hypertensive rat model. The details of this in vivo test are found in Roesler, J.M., et al., J. Pharmacol. Exp. Ther., 236:1-7 (1986). The hypotensive effects of (E)-3-[2-n-butyl-1-{(4-carboxyphenyl)methyl}-1H-imidazol-5-yl]-2-(2-thienyl)methyl}-2-propenoic acid (Compound A) before and after hydrochlorothiazide (HCTZ) administration in this test system is presented in Table 1 below. These results indicate that diuretics enhance the hypotensive efficacy of AII receptor antagonists. Thus, a combined therapy of these two classes of drugs will be likely to increase the response rate to therapy among hypertensive patients.

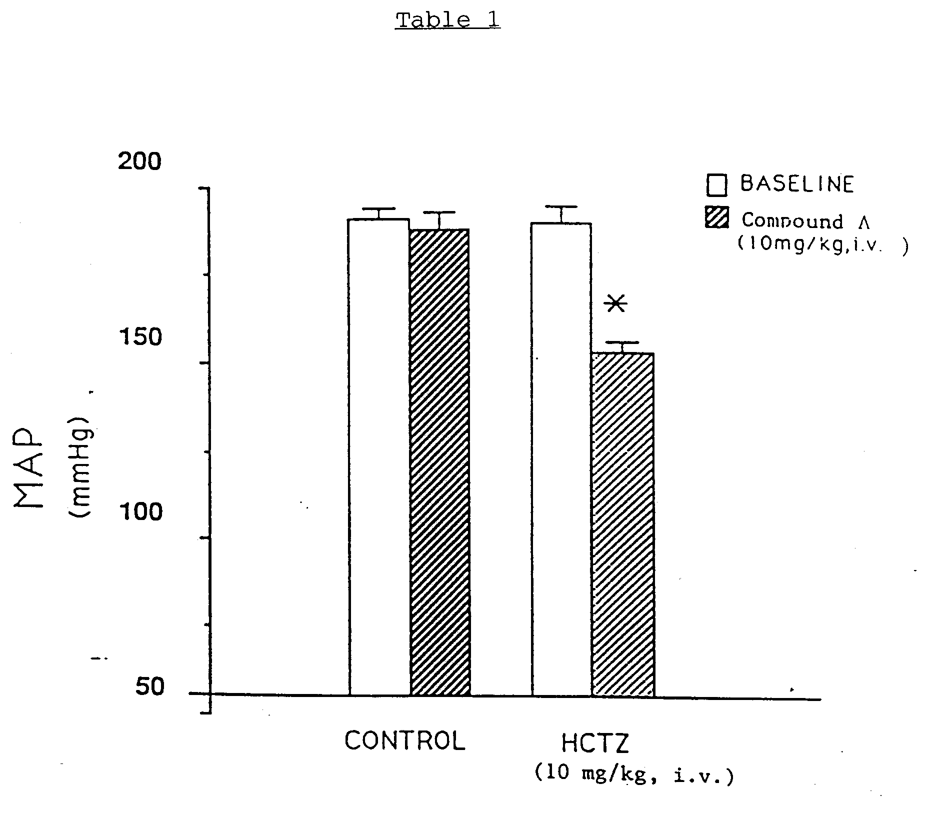

Advantageously the angiotensin II blocking compounds of formula (I) in a preparation comprising a pharmaceutical carrier, a second therapeutic agent selected from a diuretic, a calcium channel blocker, a β-adrenoceptor blocker, a renin inhibitor, or an angiotensin converting enzyme inhibitor will be present in an amount to treat hypertension in a subject in need thereof. The preparation contains the angiotensin II blocking compound in a dosage unit in an amount from about .01-200 mg, preferably 1-100 mg.

The pharmaceutical carrier may be, for example, either a solid or a liquid. The administration may be parenterally, rectally, topically, transdermally or orally, the latter being the preferred route of administration. The pharmaceutical forms are, for example, syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example, polyethylene glycol, oils, or water with a suspending agent, preservative, flavouring or colouring agent.

The present invention also provides for a controlled release formulation to be administered to a mammal comprising a mixture of a micronised AII receptor antagonist and a second agent, such as a diuretic a calcium channel blocker, a β-adrenoceptor blocker, a renin inhibitor, or an angiotensin converting enzyme inhibitor, or pharmaceutically acceptable salts thereof, a water-channeling agent and a wetting agent. The mixture is in the form of a non-compressed pellet, having an enteric coat or a sustained release coat permeable to gastrointestinal juices. These slow release pharmaceutical compositions are prepared, for example, as described in U.S. Patent Number 4,524,060, issued June 18, 1985. Other controlled release formulations are described in U.S. Patent Number 4,880,830, issued November 14, 1989 and U.S. Patent Number 5,068,112, issued November 26, 1991.

The AII receptor antagonist compounds of this invention can also be administered in combination with other antihypertensives and/or diuretics and/or angiotensin converting enzyme inhibitors and/or calcium channel blockers. For example, the compounds of this invention can be given in combination with such compounds as amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, metolazone, metoprolol tartate, methylclothiazide, methyldopa, methyldopate hydrochloride, minoxidil, pargyline hydrochloride, polythiazide, prazosin, rauwolfia serpentina, rescinnamine, reserpine, sodium nitroprusside, spironolactone, timolol maleate, trichloromethiazide, trimethophan camsylate, benzithiazide, quinethazone, ticynafan, triamterene, acetazolamide, aminophylline, cyclothiazide, ethacrynic acid, merethoxylline procaine, sodium ethacrynate, delapril hydrochloride, enalaprilat, fosinopril sodium, lisinopril, pentopril, quinapril hydrochloride, ramapril, teprotide, zofenopril calcium, diflusinal, diltiazem, felodipine nicardipine, niludipine, minodipine, nisoldipine, nitrenedipine, verapimil and the like, as well as admixtures and combinations thereof. The AII receptor antagonist compounds of this invention can also be administered in combination with a monoamine oxidase inhibitor, such as parnate.

To illustrate these combinations, one of the angiotensin II antagonists of this invention effective clinically in the 2.5-250 milligrams per day range can active ingredient in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository composition comprises a compound of the instant invention or a pharmaceutically acceptable salt thereof which is active when administered in this way, with a binding and/or lubricating agent such as polymeric glycols, gelatins or cocoa butter or other low melting vegetable or synthetic waxes or fats.

A typical transdermal formulation comprises a conventional aqueous or non-aqueous vehicle, for example, a cream, ointment lotion or paste or in the form of a medicated plaster, patch or membrane.

For topical administration, the pharmaceutical compositions adapted include solutions, suspensions, ointments, and solid inserts. Typical pharmaceutically acceptable carriers are, for example, water, mixtures of water and water-miscible solvents such as lower alkanols or vegetable oils, and water soluble ophthalomologically acceptable non-toxic polymers, for example, cellulose derivatives such as methyl cellulose. The pharmaceutical preparation may also contain non-toxic auxiliary substances such as emulsifying, preserving, wetting, and bodying agents, as for example, polyethylene glycols; antibacterial components such as quaternary ammonium compounds; buffering ingredients such as alkali metal chloride; antioxidants such as sodium metabisulfite; and other conventional ingredients such as sorbitan monolaurate.

The pharmaceutical preparations are made following conventional techniques of a pharmaceutical chemist involving mixing, granulating, and compressing, when necessary, for tablet forms, or mixing, filling and be effectively combined at levels at the 0.5-250 milligrams per day range with the following compounds at the indicated per day dose range chlorothiazide (125-2000 mg), ethacrynic acid (15-200 mg), amiloride (5-20 mg), timolol maleate (5-60 mg), methyldopa (65-2000 mg), felodipine (5-60 mg) and nitrendipine (5-60 mg). In addition, triple drug combinations of hydrochlorothiazide (15-200 mg) plus amiloride (5-20 mg) plus angiotensin II antagonist of this invention (3-200 mg) or hydrochlorothiazide (15-200 mg) plus timolol maleate (5-60 mg) plus an angiotensin II antagonist of this invention (0.5-250 mg) of hydrochlorothiazide (15-200 mg) and nifedipine (5-60 mg) plus an angiotensin II antagonist of this invention (0.5-250 mg) are effective combinations to control blood pressure in hypertensive patients. Naturally, these dose ranges can be adjusted on a unit basis as necessary to permit divided daily dosage and, as noted above, the dose will vary depending on the nature and severity of these disease, weight of patient, special diets and other factors.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

A composition for parenteral administration which can be formulated as a solution or a suspension will generally consist of a solution or suspension of the dissolving the ingredients, as appropriate, to give the desired oral, parenteral, rectal, transdermal, or topical products.

The compositions of this invention as described above are advantageously carried out in conjunction with a diuretic, particularly a thiazide diuretic, such as hydrochlorothiazide, or a loop diuretic, such as furosemide. The amount of the diuretic present in a dosage unit is from about 1 mg to about 500 mg.

Also, the compositions of this invention as described above are advantageously carried out in conjunction with a calcium channel blocker, particularly dihydropyridine calcium antagonists, such as nifedipine. The amount of the calcium channel blocker in a dosage unit is from about 5 mg to about 60 mg, preferably about 10 mg to about 30 mg.

Additionally, the compositions of this invention as described above are carried out in conjunction with a β-adrenoceptor blocker, such as propranolol. The amount of the β-adrenoceptor blocker in a dosage unit is from about 20 to about 120 mg.

Also, the compositions of this invention as described above are advantageously carried out in conjunction with a renin inhibitor, such as enalkinen. The amount of the renin inhibitor in a dosage unit is from about 10 mg to about 50 mg.

Also, the compositions of this invention as described above are advantageously carried out in conjunction with an angiotensin converting enzyme inhibitor, such as captopril or enalapril. The amount of the angiotensin converting enzyme inhibitor in a dosage unit is from about 25 to about 50 mg or from about 5 mg to about 40 mg, respectively.

The method in accordance with this invention comprises administering stepwise or in physical combination an angiotensin II receptor antagonist of formula (I) with a second agent, such as a diuretic, a calcium channel blocker, a β-adrenoceptor blocker, a renin inhibitor, or an angiotensin converting enzyme inhibitor, in an amount sufficient to treat hypertension. The active medicament preferably will be in an amount of from about 5 mg to about 250 mg. Advantageously, equal doses, or dosage units, will be administered from one to four times daily. The total daily dosage will be from about 5 mg to about 1000 mg. When the administration described above is carried out hypotensive action is achieved in hypertensive subjects in need thereof.

The following examples are not limiting but are illustrative of the preparation of certain compounds of the invention and pharmaceutical compositions containing these compounds.

### Example 1

An oral dosage form for administering orally active formula (I) compounds is produced by screening, mixing and filling into hard gelatin capsules the ingredients in proportions, for example, as shown below.

| Ingredients | Amounts |
|---|---|
| (E)-3-[2-n-butyl-1-{(4-carboxyphenyl)methyl}-1H-imidazol-5-yl]-2-(2-thienyl)-methyl-2-propenoic acid | 100 mg |
| hydrochlorothiazide | 50 mg |
| magnesium stearate | 10 mg |
| lactose | 100 mg |

### Example 2

The sucrose calcium sulfate dihydrate and orally active formula (I) compound is mixed and granulated with a 10% gelatin solution. The wet granules are screened, dried, mixed with the starch, talc and stearic acid, screened and compressed into a tablet.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 75 mg |
| hydrochlorothiazide | 40 mg |
| calcium sulfate dihydrate | 100 mg |
| sucrose | 15 mg |
| starch | 8 mg |
| talc | 4 mg |
| stearic acid | 2 mg |

### Example 3

(E)-3-[2-n-butyl-1-{(4-carboxyphenyl)methyl}-1H-imidazol-5-yl]-2-(2-thienyl)methyl-2-propenoic acid, 50 mg, and hydrochlorothiazide, 25 mg, is dispersed in 25 ml of normal saline to prepare an injectable preparation.

### Example 4

A topical ophthamological solution for administering a formula (I) compound is produced by mixing under sterile conditions the ingredients in proportions, for example, as shown below.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 1.0 mg |
| hydrochlorothiazide | 1.0 mg |
| dibasic sodium phosphate | 10.4 mg |
| monobasic sodium phosphate | 2.4 mg |
| chlorobutanol | 5.0 mg |
| hydroxypropanol methylcellulose | 5.0 mg |
| sterile water | q.s. ad 1.0 mL |
| 1.0 N sodium hydroxide | q.s. ad pH 7.4 |

### Example 5

An oral dosage form for administering an orally active formula (I) compound is produced by screening, mixing and filling into hard gelatin capsules the ingredients in proportions, for example, as shown below.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 100 mg |
| nifedipine | 10 mg |
| magnesium stearate | 10 mg |
| lactose | 100 mg |

### Example 6

The sucrose calcium sulfate dihydrate and an orally active formula (I) compound are mixed and granulated with a 10% gelatin solution. The wet granules are screened, dried, mixed with the starch, talc and stearic acid, screened and compressed into a tablet.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 75 mg |
| nifedipine | 75 mg |
| calcium sulfate dihydrate | 100 mg |
| sucrose | 15 mg |
| starch | 8 mg |
| talc | 4 mg |
| stearic acid | 2 mg |

### Example 7

(E)-3-[2-n-butyl-1-{(4-carboxyphenyl)methyl}-1H-imidazol-5-yl]-2-(2-thienyl)methyl-2-propenoic acid, 50 mg, and nifedipine, 5 mg, is dispersed in 25 ml of normal saline to prepare an injectable preparation.

### Example 8

An oral dosage form for administering orally active formula (I) - (IX) compounds is produced by screening, mixing and filling into hard gelatin capsules the ingredients in proportions, for example, as shown below.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 100 mg |
| propranolol | 100 mg |
| magnesium stearate | 10 mg |
| lactose | 100 mg |

### Example 9

The sucrose calcium sulfate dihydrate and an orally active formula (I) compound are mixed and granulated with a 10% gelatin solution. The wet granules are screened, dried, mixed with the starch, talc and stearic acids, screened and compressed into a tablet.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 75 mg |
| propranolol | 75 mg |
| calcium sulfate dihydrate | 100 mg |
| sucrose | 15 mg |
| starch | 8 mg |
| talc | 4 mg |
| stearic acid | 2 mg |

### Example 10

(E)-3-[2-n-butyl-1-{(4-carboxyphenyl)methyl}-1H-imidazol-5-yl]-2-(2-thienyl)methyl-2-propenoic acid, 50 mg, and propranolol, 50 mg, is dispersed in 25 ml of normal saline to prepare an injectable preparation.

### Example 11

A topical ophthamological solution for administering a formula (I) compound is produced by mixing under sterile conditions the ingredients in proportions, for example, as shown below.

| Ingredients | Amounts(mg/mL) |
|---|---|
| E)-3-[2-n-butyl-1-{(4-carboxyphenyl)methyl}-1H-imidazol-5-yl]-2-(2-thienyl)-methyl-2-propenoic acid | 1.0 |
| propranolol | 1.0 |
| dibasic sodium phosphate | 10.4 |
| monobasic sodium phosphate | 2.4 |
| chlorobutanol | 5.0 |
| hydroxypropanol methylcellulose | 5.0 |
| sterile water | q.s. ad 1.0 mL |
| 1.0 N sodium hydroxide | q.s. ad pH 7.4 |

### Example 12

An oral dosage form for administering an orally active formula (I) compound is produced by screening, mixing and filling into hard gelatin capsules the ingredients in proportions, for example, as shown below.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 100 mg |
| enalkinen | 50 mg |
| magnesium stearate | 10 mg |
| lactose | 100 mg |

### Example 13

The sucrose calcium sulfate dihydrate and an orally active formula (I) compound are mixed and granulated with a 10% gelatin solution. The wet granules are screened, dried, mixed with the starch, talc and stearic acid, screened and compressed into a tablet.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 75 mg |
| enalkinen | 40 mg |
| calcium sulfate dihydrate | 100 mg |
| sucrose | 15 mg |
| starch | 8 mg |
| talc | 4 mg |
| stearic acid | 2 mg |

### Example 14

(E)-3-[2-n-butyl-1-{(4-carboxyphenyl)methyl}-1H-imidazol-5-yl]-2-(2-thienyl)methyl-2-propenoic acid, 50 mg, and enalkinen, 10 mg, is dispersed in 25 ml of normal saline to prepare an injectable preparation.

### Example 15

An oral dosage form for administering an orally active formula (I) compound is produced by screening, mixing and filling into hard gelatin capsules the ingredients in proportions, for example, as shown below.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 100 mg |
| captopril | 50 mg |
| magnesium stearate | 10 mg |
| lactose | 100 mg |

### Example 16

The sucrose calcium sulfate dihydrate and an orally active formula (I) compound are mixed and granulated with a 10% gelatin solution. The wet granules are screened, dried, mixed with the starch, talc and stearic acid, screened and compressed into a tablet.

| Ingredients | Amounts |
|---|---|
| E)-3-[2-n-butyl-1-{(4-carboxyphenyl)methyl}-1H-imidazol-5-yl]-2-(2-thienyl)-methyl-2-propenoic acid | 75 mg |
| captopril | 40 mg |
| calcium sulfate dihydrate | 100 mg |
| sucrose | 15 mg |
| starch | 8 mg |
| talc | 4 mg |
| stearic acid | 2 mg |

### Example 17

(E)-3-[2-n-butyl-1-{(4-carboxyphenyl)methyl}-1H-imidazol-5-yl]-2-(2-thienyl)methyl-2-propenoic acid, 50 mg, and enalapril, 5 mg, is dispersed in 25 ml of normal saline to prepare an injectable preparation.

It is to be understood that the invention is not limited to the embodiments illustrated hereabove and the right to the illustrated embodiments and all modifications coming within the scope of the following claims is reserved.

## Claims (Claims for the following Contracting State(s): AT, BE, CH, DE, DK, FR, GB, IT, LI, LU, MC, NL, SE)

1. A pharmaceutical composition comprising a diuretic, a pharmaceutically acceptable carrier and an angiotensin II receptor antagonist of the formula (I): 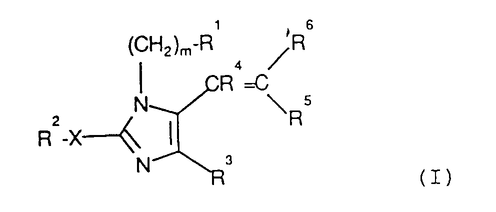 in which:
R¹ is adamantyl, phenyl, biphenyl, or naphthyl, with each aryl group being unsubstituted or substituted by one to three substituents selected from Cl, Br, F, I, C₁-C₆alkyl, nitro, A-CO₂R⁷, tetrazol-5-yl, C₁-C₆alkoxy, hydroxy, SC₁-C₆alkyl, SO₂NHR⁷, NHSO₂R⁷, SO₃H, CONR⁷R⁷, CN, SO₂C₁-C₆alkyl, PO(OR⁷)₂, NR⁷R⁷, NR⁷COH, NR⁷COC₁-C₆alkyl, NR⁷CON(R⁷)₂, NR⁷COW, W, or SO₂W;
m is 0-4;
R² is C₂-C₁₀alkyl, C₃-C₁₀alkenyl, C₃-C₁₀alkynyl, C₃-C₆cycloalkyl, or (CH₂)₀₋₈phenyl unsubstituted or substituted by one to three substituents selected from C₁-C₆alkyl, nitro, Cl, Br, F, I, hydroxy, C₁-C₆alkoxy, NR⁷R⁷, CO₂R⁷, CN, CONR⁷R⁷, W, tetrazol-5-yl, NR⁷COC₁-C₆alkyl, NR⁷COW, SC₁-C₆alkyl, SO₂W, or SO₂C₁-C₆alkyl;
X is a single bond, S, NR⁷, or O;
R³ is hydrogen, Cl, Br, F, I, CHO, hydroxymethyl, COOR⁷, CONR⁷R⁷, NO₂, W, CN, NR⁷R⁷, or phenyl;
R⁴ and R⁵ are independently hydrogen, C₁-C₆alkyl, thienyl-Y-, furyl-Y-, pyrazolyl-Y-, imidazolyl-Y-, pyrrolyl-Y-, triazolyl-Y-, oxazolyl-Y-, isoxazolyl-Y-, thiazolyl-Y-, pyridyl-Y-, or tetrazolyl-Y-, except that R⁴ and R⁵ are not both selected from hydrogen and C₁-C₆alkyl and each heterocyclic ring is unbsubstituted or substituted by C₁-C₆alkyl, C₁-C₆alkoxy,Cl, Br, F, I, NR⁷R⁷, CO₂R⁷, SO₂NHR⁷, SO₃H, CONR²R⁷, OH, NO₂, W, SO₂W, SC₁-C₆alkyl, SO₂-C₁-C₆alkyl, NR⁷COH, NR⁷COW, or NR⁷COC₁-C₆alkyl;
Y is a single bond, O, S, or C₁-C₆alkyl which is straight or branched or optionally substituted by phenyl or benzyl, wherein each of the aryl groups is unsubstituted or substituted by halo, NO₂, CF₃, C₁-C₆alkyl, C₁-C₆alkoxy, CN, or CO₂R⁷;
R⁶ is -Z-COOR⁸ or -Z-CONR⁷R⁷;
Z is a single bond, vinyl, -CH₂-O-CH₂-, methylene optionally substituted by C₁-C₆alkyl, one or two benzyl groups, thienylmethyl, or furylmethyl, or -C(O)NHCR⁹-, wherein R⁹ is H, C₁-C₆alkyl, phenyl, benzyl, thienylmethyl, or furylmethyl;
W is CₙF₂ₙ₊₁, wherein n is 1-3;
A is -(CH₂)ₘ-, -CH=CH-, -O(CH₂)ₙ-, or -S(CH₂)ₙ-;
each R⁷ independently is hydrogen, C₁-C₆alkyl, or (CH₂)ₘphenyl, where m is 0-4; and
R⁸ is hydrogen, C₁-C₆alkyl, or 2-di(C₁-C₆alkyl)amino-2-oxoethyl;
or a pharmaceutically acceptable salt thereof.

2. The pharmaceutical composition according to claim 1 wherein the diuretic is hydrochlorothiazide or furosemide.

3. A pharmaceutical composition comprising a calcium channel blocker, a pharmaceutically acceptable carrier and an angiotensin II receptor antagonist of the formula (I), as described in claim 1.

4. The pharmaceutical composition according to claim 3 wherein the calcium channel blocker is nifedipine, diltiazem, or verapimil.

5. A pharmaceutical composition comprising a β-adrenoceptor blocker, a pharmaceutically acceptable carrier and an angiotensin II receptor antagonist of the formula (I), as described in claim 1.

6. The pharmaceutical composition according to claim 5 wherein the β-adrenoceptor blocker is propranolol.

7. A pharmaceutical composition comprising a renin inhibitor, a pharmaceutically acceptable carrier and an angiotensin II receptor antagonist of the formula (I), as described in claim 1.

8. The pharmaceutical composition according to claim 7 wherein the renin inhibitor is enalkinen.

9. A pharmaceutical composition comprising an angiotensin converting enzyme inhibitor, a pharmaceutically acceptable carrier and an angiotensin II receptor antagonist of the formula (I), as described in claim 1.

10. The pharmaceutical composition according to claim 9 wherein the angiotensin converting enzyme inhibitor is captopril or enalapril.

11. The pharmaceutical composition according to any one of claims 1 to 10 wherein the angiotensin II receptor antagonist is (E)-3-[2-n-butyl-1-{(4-carboxyphenyl)methyl}-1H-imidazol-5-yl]-2-(2-thienyl)methyl-2-propenoic acid or a pharmaceutically acceptable salt thereof.

12. The pharmaceutical composition according to anyone of claims 1 to 10 wherein the angiotensin II receptor antagonist is (E)-3-[2-n-butyl-1-{(4-carboxyphenyl)methyl}-1H-imidazol-5-yl]-2-(2-thienyl) methyl-2-propenoic acid methanesulfonate.

13. The pharmaceutical composition according to any one of claims 1 to 10 wherein the angiotensin II receptor antagonist is (E)-3-[2-n-butyl-1-{(4-carboxynapth-1-yl)methyl}-1H-imidazol-5-yl]-2-(2-thienyl)methyl-2-propenoic acid or a pharmaceutically acceptable salt thereof.

14. The use of an angiotensin II receptor antagonist of the formula (I), as described in claim 1, and a diuretic in the manufacture of a medicament for the treatment of hypertension.

15. The use according to claim 14 wherein the diuretic is hydrochlorothiazide or furosemide.

16. The use of an angiotensin II receptor antagonist of the formula (I), as described in claim 1, and a calcium channel blocker in the manufacture of a medicament for the treatment of hypertension.

17. The use according to claim 16 wherein the calcium channel blocker is nifedipine, diltiazem, or verapimil.

18. The use of an angiotensin II receptor antagonist of the formula (I), as described in claim 1, and a β-adrenoceptor blocker in the manufacture of a medicament for the treatment of hypertension.

19. The use according to claim 18 wherein the β-adrenoceptor blocker is propranolol.

20. The use of an angiotensin II receptor antagonist of the formula (I), as described in claim 1, and a renin inhibitor in the manufacture of a medicament for the treatment of hypertension.

21. The use according to claim 20 wherein the renin inhibitor is enalkinen.

22. The use of an angiotensin II receptor antagonist of the formula (I), as described in claim 1, and an angiotensin converting enzyme inhibitor in the manufacture of a medicament for the treatment of hypertension.

23. The use according to claim 22 wherein the angiotensin converting enzyme inhibitor is captopril or enalapril.

24. The use according to any one of claims 14 to 23 wherein the angiotensin II receptor antagonist is (E)-3-[2-n-butyl-1-{(4-carboxyphenyl)methyl}-1H-imidazol-5-yl]-2-(2-thienyl)-methyl-2-propenoic acid or a pharmaceutically acceptable salt thereof.

25. The use according to any one of claims 14 to 23 wherein the angiotensin II receptor antagonist is (E)-3-[2-n-butyl-1-{(4-carboxyphenyl)methyl}-1H-imidazol-5-yl]-2-(2-thienyl)-methyl-2-propenoic acid methanesulfonate.

26. The use according to any one of claims 14 to 23 wherein the angiotensin II receptor antagonist is (E)-3-[2-n-butyl-1-{(4-carboxynapth-1-yl)methyl}-1H-imidazol-5-yl]-2-(2-thienyl)methyl-2-propenoic acid or a pharmaceutically acceptable salt thereof.

## Claims (Claims for the following Contracting State(s): ES, GR)

1. A process for preparing a pharmaceutical composition which comprises bringing into association a diuretic, a pharmaceutically acceptable carrier and an angiotensin II receptor antagonist of the formula (I): 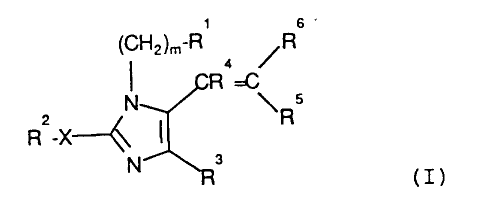 in which:
R¹ is adamantyl, phenyl, biphenyl, or naphthyl, with each aryl group being unsubstituted or substituted by one to three substituents selected from Cl, Br, F, I, C₁-C₆alkyl, nitro, A-CO₂R⁷, tetrazol-5-yl, C₁-C₆alkoxy, hydroxy, SC₁-C₆alkyl, SO₂NHR⁷, NHSO₂R⁷, SO₃H, CONR⁷R⁷, CN, SO₂C₁-C₆alkyl, PO(OR⁷)₂, NR⁷R⁷, NR⁷COH, NR⁷COC₁-C₆alkyl, NR⁷CON(R⁷)₂, NR⁷COW, W, or SO₂W;
m is 0-4;
R² is C₂-C₁₀alkyl, C₃-C₁₀alkenyl, C₃-C₁₀alkynyl, C₃-C₆cycloalkyl, or (CH₂)₀₋₈phenyl unsubstituted or substituted by one to three substituents selected from C₁-C₆alkyl, nitro, Cl, Br, F, I, hydroxy, C₁-C₆alkoxy, NR⁷R⁷, CO₂R⁷, CN, CONR⁷R⁷, W, tetrazol-5-yl, NR⁷COC₁-C₆alkyl, NR⁷COW, SC₁-C₆alkyl, SO₂W, or SO₂C₁-C₆alkyl;
X is a single bond, S, NR⁷, or O;
R³ is hydrogen, Cl, Br, F, I, CHO, hydroxymethyl, COOR⁷, CONR⁷R⁷, NO₂, W, CN, NR⁷R⁷, or phenyl;
R⁴ and R⁵ are independently hydrogen, C₁-C₆alkyl, thienyl-Y-, furyl-Y-, pyrazolyl-Y-, imidazolyl-Y-, pyrrolyl-Y-, triazolyl-Y-, oxazolyl-Y-, isoxazolyl-Y-, thiazolyl-Y-, pyridyl-Y-, or tetrazolyl-Y-, except that R⁴ and R⁵ are not both selected from hydrogen and C₁-C₆alkyl and each heterocyclic ring is unbsubstituted or substituted by C₁-C₆alkyl, C₁-C₆alkoxy,Cl, Br, F, I, NR⁷R⁷, CO₂R⁷, SO₂NHR⁷, SO₃H, CONR²R⁷, OH, NO₂, W, SO₂W, SC₁-C₆alkyl, SO₂-C₁-C₆alkyl, NR⁷COH, NR⁷COW, or NR⁷COC₁-C₆alkyl;
Y is a single bond, O, S, or C₁-C₆alkyl which is straight or branched or optionally substituted by phenyl or benzyl, wherein each of the aryl groups is unsubstituted or substituted by halo, NO₂, CF₃, C₁-C₆alkyl, C₁-C₆alkoxy, CN, or CO₂R⁷;
R⁶ is -Z-COOR⁸ or -Z-CONR⁷R⁷;
Z is a single bond, vinyl, -CH₂-O-CH₂-, methylene optionally substituted by C₁-C₆alkyl, one or two benzyl groups, thienylmethyl, or furylmethyl, or -C(O)NHCR⁹-, wherein R⁹ is H, C₁-C₆alkyl, phenyl, benzyl, thienylmethyl, or furylmethyl;
W is CₙF₂ₙ₊₁, wherein n is 1-3;
A is -(CH₂)ₘ-, -CH=CH-, -O(CH₂)ₙ-, or -S(CH₂)ₙ-;
each R⁷ independently is hydrogen, C₁-C₆alkyl, or (CH₂)ₘphenyl, where m is 0-4; and
R⁸ is hydrogen, C₁-C₆alkyl, or 2-di(C₁-C₆alkyl)amino-2-oxoethyl;
or a pharmaceutically acceptable salt thereof.

2. The process according to claim 1 wherein the diuretic is hydrochlorothiazide or furosemide.

3. A process for preparing a pharmaceutical composition which comprises bringing into association a calcium channel blocker, a pharmaceutically acceptable carrier and an angiotensin II receptor antagonist of the formula (I), as described in claim 1.

4. The process according to claim 3 wherein the calcium channel blocker is nifedipine, diltiazem, or verapimil.

5. A process for preparing a pharmaceutical composition which comprises bringing into association a β-adrenoceptor blocker, a pharmaceutically acceptable carrier and an angiotensin II receptor antagonist of the formula (I), as described in claim 1.

6. The process according to claim 5 wherein the β-adrenoceptor blocker is propranolol.

7. A process for preparing a pharmaceutical composition which comprises bringing into association a renin inhibitor, a pharmaceutically acceptable carrier and an angiotensin II receptor antagonist of the formula (I), as described in claim 1.

8. The process according to claim 7 wherein the renin inhibitor is enalkinen.

9. A process for preparing a pharmaceutical composition which comprises bringing into association an angiotensin converting enzyme inhibitor, a pharmaceutically acceptable carrier and an angiotensin II receptor antagonist of the formula (I), as described in claim 1.

10. The process according to claim 9 wherein the angiotensin converting enzyme inhibitor is captopril or enalapril.

11. The process according to any one of claims 1 to 10 wherein the angiotensin II receptor antagonist is (E)-3-[2-n-butyl-1-{(4-carboxyphenyl)methyl}-1H-imidazol-5-yl]-2-(2-thienyl)-methyl-2-propenoic acid or a pharmaceutically acceptable salt thereof.

12. The process according to any one of claims 1 to 10 wherein the angiotensin II receptor antagonist is(E)-3-[2-n-butyl-1-{(4-carboxyphenyl)methyl}-1H-imidazol-5-yl]-2-(2-thienyl)-methyl-2-propenoic acid methanesulfonate.

13. The process according to any one of claims 1 to 10 wherein the angiotensin II receptor antagonist is (E)-3-[2-n-butyl-1-{(4-carboxynapth-1-yl)methyl}-1H-imidazol-5-yl]-2-(2-thienyl)methyl-2-propenoic acid or a pharmaceutically acceptable salt thereof.

14. The use of an angiotensin II receptor antagonist of the formula (I), as described in claim 1, and a diuretic in the manufacture of a medicament for the treatment of hypertension.

15. The use according to claim 14 wherein the diuretic is hydrochlorothiazide or furosemide.

16. The use of an angiotensin II receptor antagonist of the formula (I), as described in claim 1, and a calcium channel blocker in the manufacture of a medicament for the treatment of hypertension.

17. The use according to claim 16 wherein the calcium channel blocker is nifedipine, diltiazem, or verapimil.

18. The use of an angiotensin II receptor antagonist of the formula (I), as described in claim 1, and a β-adrenoceptor blocker in the manufacture of a medicament for the treatment of hypertension.

19. The use according to claim 18 wherein the β-adrenoceptor blocker is propranolol.

20. The use of an angiotensin II receptor antagonist of the formula (I), as described in claim 1, and a renin inhibitor in the manufacture of a medicament for the treatment of hypertension.

21. The use according to claim 20 wherein the renin inhibitor is enalkinen.

22. The use of an angiotensin II receptor antagonist of the formula (I), as described in claim 1, and an angiotensin converting enzyme inhibitor in the manufacture of a medicament for the treatment of hypertension.

23. The use according to claim 22 wherein the angiotensin converting enzyme inhibitor is captopril or enalapril.

24. The use according to any one of claims 14 to 23 wherein the angiotensin II receptor antagonist is (E)-3-[2-n-butyl-1-{(4-carboxyphenyl)methyl}-1H-imidazol-5-yl]-2-(2-thienyl)-methyl-2-propenoic acid or a pharmaceutically acceptable salt thereof.

25. The use according to any one of claims 14 to 23 wherein the angiotensin II receptor antagonist is (E)-3-[2-n-butyl-1-{(4-carboxyphenyl)methyl}-1H-imidazol-5-yl]-2-(2-thienyl)-methyl-2-propenoic acid methanesulfonate.

26. The use according to any one of claims 14 to 23 wherein the angiotensin II receptor antagonist is (E)-3-[2-n-butyl-1-{(4-carboxynapth-1-yl)methyl}-1H-imidazol-5-yl]-2-(2-thienyl)methyl-2-propenoic acid or a pharmaceutically acceptable salt thereof.

27. A pharmaceutical composition comprising a diuretic, a pharmaceutically acceptable carrier and an angiotensin II receptor antagonist of the formula (I): 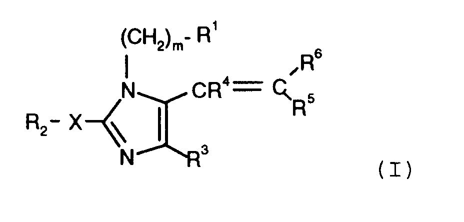 in which:
R¹ is adamantyl, phenyl, biphenyl, or naphthyl, with each aryl group being unsubstituted or substituted by one to three substituents selected from Cl, Br, F, I, C₁-C₆ alkyl, nitro, A-CO₂R⁷, tetrazol-5-yl, C₁-C₆alkoxy, hydroxy, SC₁-C₆alkyl, SO₂NHR⁷, NHSO₂R⁷, SO₃H, CONR⁷R⁷, CN, SO₂C₁-C₆alkyl, NHSO₂R⁷, PO(OR⁷)₂, NR⁷R⁷, NR⁷COH, NR⁷COC₁-C₆alkyl, NR⁷CON(R⁷)₂, NR⁷COW, W, or SO₂W;
m is 0-4;
R² is C₂-C₁₀alkyl, C₃-C₁₀alkenyl, C₃-C₁₀alkynyl, C₁-C₆cycloalkyl, or (CH₂)₀₋₈phenyl unsubstituted or substituted by one to three substituents selected from C₁-C₆alkyl, nitro, Cl, Br, F, I, hydroxy, C₁-C₆alkoxy, NR⁷R⁷, CO₂R⁷, CN, CONR⁷R⁷, W, tetrazol-5-yl, NR⁷COC₁-C₆alkyl, NR⁷COW, SC₁-C₆alkyl, SO₂W, or SO₂C₁-C₆alkyl;
X is a single bond, S, NR⁷, or O;
R³ is hydrogen, Cl, Br, F, I, CHO, hydroxymethyl, COOR⁷, CONR⁷R⁷,NO₂, W, CN, NR⁷R⁷, or phenyl;
R⁴ and R⁵ are independently hydrogen, C₁-C₆alkyl, thienyl-Y-, furyl-Y-, pyrazolyl-Y-, imidazolyl-Y-pyrrolyl-Y-, triazolyl-Y-, oxazolyl-Y-, isoxazolyl-Y-, thiazolyl-Y-, pyridyl-Y-, or tetrazolyl-Y-, except that R⁴ and R⁵ are not both selected from hydrogen and C₁-C₆alkyl and each heterocyclic ring is unbsubstituted or substituted by C₁-C₆alkyl, C₁-C₆alkoxy,Cl, Br, F, I, NR⁷R⁷, CO₂R⁷, SO₂NHR⁷, SO₃H, CONR²R⁷, OH, NO₂, W, SO₂W, SC₁-C₆alkyl, SO₂-C₁-C₆alkyl, NR⁷COH, NR⁷COW, or NR⁷COC₁-C₆alkyl;
Y is a single bond, O, S, or C₁-C₆alkyl which is straight or branched or optionally substituted by phenyl or benzyl, wherein each of the aryl groups is unsubstituted or substituted by halo, NO₂, CF₃, C₁-C₆alkyl, C₁-C₆alkoxy, CN, or CO₂R⁷;
R⁶ is -Z-COOR⁸ or -Z-CONR⁷R⁷;
Z is a single bond, vinyl, -CH₂-O-CH₂-, methylene optionally substituted by C₁-C₆alkyl, one or two benzyl groups, thienylmethyl, or furylmethyl, or -C(O)NHCR⁹-, wherein R⁹ is H, C₁-C₆alkyl, phenyl, benzyl, thienylmethyl, or furylmethyl;
W is CₙF₂ₙ₊₁, wherein n is 1-3;
A is -(CH₂)ₘ-, -CH=CH-, -O(CH₂)ₙ-, or -S(CH₂)ₙ-;
each R⁷ independently is hydrogen, C₁-C₆alkyl, or (CH₂)ₘphenyl, where m is 0-4; and
R⁸ is hydrogen, C₁-C₆alkyl, or 2-di(C₁-C₆alkyl)amino-2-oxoethyl;
or a pharmaceutically acceptable salt thereof.

28. The pharmaceutical composition according to claim 27 wherein the diuretic is hydrochlorothiazide or furosemide.

29. A pharmaceutical composition comprising a calcium channel blocker, a pharmaceutically acceptable carrier and an angiotensin II receptor antagonist of the formula (I), as described in claim 27.

30. The pharmaceutical composition according to claim 29 wherein the calcium channel blocker is nifedipine, diltiazem, or verapimil.

31. A pharmaceutical composition comprising a β-adrenoceptor blocker, a pharmaceutically acceptable carrier and an angiotensin II receptor antagonist of the formula (I), as described in claim 27.

32. The pharmaceutical composition according to claim 31 wherein the β-adrenoceptor blocker is propranolol.

33. A pharmaceutical composition comprising a renin inhibitor, a pharmaceutically acceptable carrier and an angiotensin II receptor antagonist of the formula (I), as described in claim 27.

34. The pharmaceutical composition according to claim 33 wherein the renin inhibitor is enalkinen.

35. A pharmaceutical composition comprising an angiotensin converting enzyme inhibitor, a pharmaceutically acceptable carrier and an angiotensin II receptor antagonist of the formula (I), as described in claim 27.

36. The pharmaceutical composition according to claim 35 wherein the angiotensin converting enzyme inhibitor is captopril or enalapril.

37. The pharmaceutical composition according to any one of claims 27 to 36 wherein the angiotensin II receptor antagonist is (E)-3-[2-n-butyl-1-{(4-carboxyphenyl)methyl}-1H-imidazol-5-yl]-2-(2-thienyl)methyl-2-propenoic acid or a pharmaceutically acceptable salt thereof.

38. The pharmaceutical composition according to any one of claims 27 to 36 wherein the angiotensin II receptor antagonist is (E)-3-[2-n-butyl-1-{(4-carboxyphenyl)methyl}-1H-imidazol-5-yl]-2-(2-thienyl)methyl-2-propenoic acid methanesulfonate.

39. The pharmaceutical composition according to any one of claims 27 to 36 wherein the angiotensin II receptor antagonist is (E)-3-[2-n-butyl-1-{(4-carboxynapth-1-yl)methyl}-1H-imidazol-5-yl]-2-(2-thienyl)methyl-2-propenoic acid or a pharmaceutically acceptable salt thereof.

## Patentansprüche (Patentansprüche für folgende(n) Vertragsstaat(en): AT, BE, CH, DE, DK, FR, GB, IT, LI, LU, MC, NL, SE)

1. Arzneimittelzusammensetzung umfassend ein Diuretikum, einen pharmazeutisch verträglichen Träger und einen Angiotensin-II-Rezeptorantagonisten der Formel (I): in der 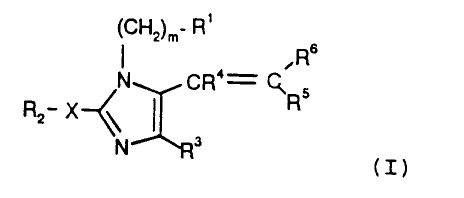
R¹ ein Adamantyl-, Phenyl-, Biphenyl- oder Naphthylrest ist, wobei jeweils der Arylrest unsubstituiert oder mit einem bis drei Substituenten substituiert ist, ausgewählt aus Cl, Br, F, I, einem C₁₋₆-Alkylrest, einer Nitrogruppe, einem Rest A-CO₂R⁷, Tetrazol-5-yl, einem C₁₋₆-Alkoxyrest, einer Hydroxygruppe, einem SC₁₋₆-Alkylrest, SO₂NHR⁷, NHSO₂R⁷, SO₃H, CONR⁷R⁷, CN, einem SO₂C₁₋₆-Alkylrest, PO(OR⁷)₂, NR⁷R⁷, NR⁷COH, einem NR⁷COC₁₋₆-Alkylrest, NR⁷CON(R⁷)₂, NR⁷COW, W oder SO₂W;
m 0 bis 4 ist;
R² ein C₂₋₁₀-Alkylrest, ein C₃₋₁₀-Alkenylrest, ein C₃₋₁₀-Alkynylrest, ein C₃₋₆-Cycloalkylrest oder ein (CH₂)₀₋₈-Phenylrest ist, der unsubstituiert oder mit einem bis drei Substituenten substituiert ist, ausgewählt aus einem C₁₋₆-Alkylrest, einer Nitrogruppe, Cl, Br, F, I, einer Hydroxygruppe, einem C₁₋₆-Alkoxyrest, NR⁷R⁷, CO₂R⁷, CN, CONR⁷R⁷, W, Tetrazol-5-yl, einem NR⁷COC₁₋₆-Alkylrest, NR⁷COW, einem SC₁₋₆-Alkylrest, SO₂W oder einem SO₂C₁₋₆-Alkylrest;
X eine Einfachbindung, S, NR⁷ oder O ist;
R³ ein Wasserstoffatom, Cl, Br, F, I, CHO, eine Hydroxymethylgruppe, COOR⁷, CONR⁷R⁷, NO₂, W, CN, NR⁷R⁷ oder ein Phenylrest ist;
R⁴ und R⁵ unabhängig voneinander ein Wasserstoffatom, ein C₁₋₆-Alkylrest, eine Thienyl-Y-, Furyl-Y-, Pyrazolyl-Y-, Imidazolyl-Y-, Pyrrolyl-Y-, Triazolyl-Y-, Oxazolyl-Y-, Isoxazolyl-Y-, Thiazolyl-Y-, Pyridyl-Y- oder Tetrazolyl-Y-Gruppe ist, mit der Ausnahme, daß R⁴ und R⁵ nicht beide ausgewählt sind aus einem Wasserstoffatom und einem C₁₋₆-Alkylrest und jeder heterocyclische Ring unsubstituiert oder mit einem C₁₋₆-Alkylrest, einem C₁₋₆-Alkoxyrest, Cl, Br, F, I, NR⁷R⁷, CO₂R⁷, SO₂NHR⁷, SO₃H, CONR²R⁷, OH, NO₂, W, SO₂W, einem SC₁₋₆-Alkylrest, einem SO₂C₁₋₆-Alkylrest, NR⁷COH, NR⁷COW oder einem NR⁷COC₁₋₆-Alkylrest substituiert ist;
Y eine Einfachbindung, O, S oder ein unverzweigter oder verzweigter C₁₋₆-Alkylrest ist, der gegebenenfalls mit Phenyl oder Benzyl substituiert ist, wobei jeder der Arylreste unsubstituiert oder mit einem Halogenatom, NO₂, CF₃, einem C₁₋₆-Alkylrest, einem C₁₋₆-Alkoxyrest, CN oder CO₂R⁷ substituiert ist;
R⁶ -Z-COOR⁸ oder -Z-CONR⁷R⁷ ist;
Z eine Einfachbindung, eine Vinylgruppe, -CH₂-O-CH₂-, eine gegebenenfalls mit einem C₁₋₆-Alkylrest, einer oder zwei Benzylgruppen, einer Thienylmethyl- oder einer Furylmethylgruppe substituierte Methylengruppe, -C(O)NHCR⁹-, wobei R⁹ ein Wasserstoffatom ist, ein C₁₋₆-Alkylrest, eine Phenyl-, Benzyl-, Thienylmethyl- oder Furylmethylgruppe ist;
W CₙF₂ₙ₊₁ ist, wobei n 1 bis 3 ist;
A -(CH₂)ₘ-, -CH=CH-, -O(CH₂)ₙ- oder -S(CH₂)ₙ- ist;
jeder Rest R⁷ unabhängig ein Wasserstoffatom, ein C₁₋₆-Alkylrest oder eine (CH₂)ₘ-Phenylgruppe ist, wobei m 0 bis 4 ist; und
R⁸ ein Wasserstoffatom, ein C₁₋₆-Alkylrest, oder 2-Di(C₁₋₆-alkyl)-amino-2-oxoethyl ist;
oder ein pharmazeutisch verträgliches Salz davon.

2. Arzneimittelzusammensetzung nach Anspruch 1, wobei das Diuretikum Hydrochlorthiazid oder Furosemid ist.

3. Arzneimittelzusammensetzung umfassend einen Calciumkanalblocker, einen pharmazeutisch verträglichen Träger und einen Angiotensin-II-Rezeptorantagonisten der Formel (I), wie in Anspruch 1 beschrieben.

4. Arzneimittelzusammensetzung nach Anspruch 3, wobei der Calciumkanalblocker Nifedipin, Diltiazem oder Verapimil ist.

5. Arzneimittelzusammensetzung umfassend einen β-Adrenorezeptorblocker, einen pharmazeutisch verträglichen Träger und einen Angiotensin-II-Rezeptorantagonisten der Formel (I) wie in Anspruch 1 beschrieben.

6. Arzneimittelzusammensetzung nach Anspruch 5, wobei der β-Adrenorezeptorblocker Propanolol ist.

7. Arzneimittelzusammensetzung umfassend einen Renininhibitor, einen pharmazeutisch verträglichen Träger und einen Angiotensin-II-Rezeptorantagonisten der Formel (I), wie in Anspruch 1 beschrieben.

8. Arzneimittelzusammensetzung nach Anspruch 7, wobei der Renininhibitor Enalkinen ist.

9. Arzneimittelzusammensetzung umfassend einen Inhibitor des Enzyms, das Angiotensin umsetzt, einen pharmazeutisch verträglichen Träger und einen Angiotensin-II-Rezeptorantagonisten der Formel (I), wie in Anspruch 1 beschrieben.

10. Arzneimittelzusammensetzung nach Anspruch 9, wobei der Inhibitor des Enzyms, das Angiotensin umsetzt, Captopril oder Enalapril ist.

11. Arzneimittelzusammensetzung nach einem der Ansprüche 1 bis 10, wobei der Angiotensin-II-Rezeptorantagonist (E)-3-[2-n-butyl-1-{(4-carboxyphenyl)methyl}-1H-imidazol-5-yl]-2-(2-thienyl)-methyl-2-propensäure ist oder ein pharmazeutisch verträgliches Salz davon.

12. Arzneimittelzusammensetzung nach einem der Ansprüche 1 bis 10, wobei der Angiotensin-II-Rezeptorantagonist (E)-3-[2-n-butyl-1-{(4-carboxyphenyl)methyl}-1H-imidazol-5-yl]-2-(2-thienyl)-methyl-2-propensäuremethansulfonat ist.

13. Arzneimittelzusammensetzung nach einem der Ansprüche 1 bis 10, wobei der Angiotensin-II-Rezeptorantagonist (E)-3-[2-n-butyl-1-{(4-carboxynaphth-1-yl)methyl}-1H-imidazol-5-yl]-2-(2-thienyl)-methyl-2-propensäure ist oder ein pharmazeutisch verträgliches Salz davon.

14. Verwendung eines Angiotensin-II-Rezeptorantagonisten der Formel (I), wie in Anspruch 1 beschrieben, und eines Diuretikums zur Herstellung eines Arzneimittels zur Behandlung von Bluthochdruck.

15. Verwendung nach Anspruch 14, wobei das Diuretikum Hydrochlorthiazid oder Furosemid ist.

16. Verwendung eines Angiotensin-II-Rezeptorantagonisten der Formel (I), wie in Anspruch 1 beschrieben, und eines Calciumkanalblockers zur Herstellung eines Arzneimittels zur Behandlung von Bluthochdruck.

17. Verwendung nach Anspruch 16, wobei der Calciumkanalblocker Nifedipin, Diltiazem oder Verapimil ist.

18. Verwendung eines Angiotensin-II-Rezeptorantagonisten der Formel (I), wie in Anspruch 1 beschrieben, und eines β-Adrenorezeptorblockers zur Herstellung eines Arzneimittels zur Behandlung von Bluthochdruck.

19. Verwendung nach Anspruch 18, wobei der β-Adrenorezeptorblocker Propanolol ist.

20. Verwendung eines Angiotensin-II-Rezeptorantagonisten der Formel (I), wie in Anspruch 1 beschrieben, und eines Renininhibitors zur Herstellung eines Arzneimittels zur Behandlung von Bluthochdruck.

21. Verwendung nach Anspruch 20, wobei der Renininhibitor Enalkinen ist.

22. Verwendung eines Angiotensin-II-Rezeptorantagonisten der Formel (I) wie in Anspruch 1 beschrieben und eines Inhibitors des Enzyms, das Angiotensin umsetzt, zur Herstellung eines Arzneimittels zur Behandlung von Bluthochdruck.

23. Verwendung nach Anspruch 22, wobei der Inhibitor des Enzyms, das Angiotensin umsetzt, Captopril oder Enalapril ist.

24. Verwendung nach einem der Ansprüche 14 bis 23, wobei der Angiotensin-II-Rezeptorantagonist (E)-3-[2-n-butyl-1-{(4-carboxyphenyl)methyl}-1H-imidazol-5-yl]-2-(2-thienyl)methyl-2-propensäure ist oder ein pharmazeutisch verträgliches Salz davon.

25. Verwendung nach einem der Ansprüche 14 bis 23, wobei der Angiotensin-II-Rezeptorantagonist (E)-3-[2-n-butyl-1-{(4-carboxyphenyl)methyl}-1H-imidazol-5-yl]-2-(2-thienyl)methyl-2-propensäuremethansulfonat ist.

26. Verwendung nach einem der Ansprüche 14 bis 23, wobei der Angiotensin-II-Rezeptorantagonist (E)-3-[2-n-butyl-1-{(4-carboxynaphth-1-yl)methyl}-1H-imidazol-5-yl]-2-(2-thienyl)-methyl-2-propensäure ist oder ein pharmazeutisch verträgliches Salz davon.

## Patentansprüche (Patentansprüche für folgende(n) Vertragsstaat(en): ES, GR)

1. Verfahren zur Herstellung einer Arzneimittelzusammensetzung umfassend das Zusammenbringen eines Diuretikums, eines pharmazeutisch verträglichen Trägers und eines Angiotensin-II-Rezeptorantagonisten der Formel (I): in der 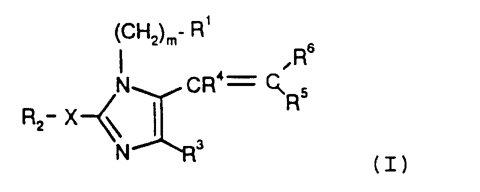
R¹ ein Adamantyl-, Phenyl-, Biphenyl- oder Naphthylrest ist, wobei jeweils der Arylrest unsubstituiert oder mit einem bis drei Substituenten substituiert ist, ausgewählt aus Cl, Br, F, I, einem C₁₋₆-Alkylrest, einer Nitrogruppe, einem Rest A-CO₂R⁷, Tetrazol-5-yl, einem C₁₋₆-Alkoxyrest, einer Hydroxygruppe, einem SC₁₋₆-Alkylrest, SO₂NHR⁷, NHSO₂R⁷, SO₃H, CONR⁷R⁷, CN, einem SO₂C₁₋₆-Alkylrest, PO(OR⁷)₂, NR⁷R⁷, NR⁷COH, einem NR⁷COC₁₋₆-Alkylrest, NR⁷CON(R⁷)₂, NR⁷COW, W oder SO₂W;
m 0 bis 4 ist;
R² ein C₂₋₁₀-Alkylrest, ein C₃₋₁₀-Alkenylrest, ein C₃₋₁₀-Alkynylrest, ein C₃₋₆-Cycloalkylrest oder ein (CH₂)₀₋₈-Phenylrest ist, der unsubstituiert oder mit einem bis drei Substituenten substituiert ist, ausgewählt aus einem C₁₋₆-Alkylrest, einer Nitrogruppe, Cl, Br, F, I, einer Hydroxygruppe, einem C₁₋₆-Alkoxyrest, NR⁷R⁷, CO₂R⁷, CN, CONR⁷R⁷, W, Tetrazol-5-yl, einem NR⁷COC₁₋₆-Alkylrest, NR⁷COW, einem SC₁₋₆-Alkylrest, SO₂W oder einem SO₂C₁₋₆-Alkylrest;
X eine Einfachbindung, S, NR⁷ oder O ist; R³ ein Wasserstoffatom, Cl, Br, F, I, CHO, eine Hydroxymethylgruppe, COOR⁷, CONR⁷R⁷, NO₂, W, CN, NR⁷R⁷ oder ein Phenylrest ist;
R⁴ und R⁵ unabhängig voneinander ein Wasserstoffatom, ein C₁₋₆-Alkylrest, eine Thienyl-Y-, Furyl-Y-, Pyrazolyl-Y-, Imidazolyl-Y-, Ryrrolyl-Y-, Triazolyl-Y-, Oxazolyl-Y-, Isoxazolyl-Y-, Thiazolyl-Y-, Pyridyl-Y- oder Tetrazolyl-Y-Gruppe ist, mit der Ausnahme, daß R⁴ und R⁵ nicht beide ausgewählt sind aus einem Wasserstoffatom und einem C₁₋₆-Alkylrest und jeder heterocyclische Ring unsubstituiert oder mit einem C₁₋₆-Alkylrest, einem C₁₋₆-Alkoxyrest, Cl, Br, F, I, NR⁷R⁷, CO₂R⁷, SO₂NHR⁷, SO₃H, CONR²R⁷, OH, NO₂, W, SO₂W, einem SC₁₋₆-Alkylrest, einem SO₂C₁₋₆-Alkylrest, NR⁷COH, NR⁷COW oder einem NR⁷COC₁₋₆-Alkylrest substituiert ist;
Y eine Einfachbindung, O, S oder ein unverzweigter oder verzweigter C₁₋₆-Alkylrest ist, der gegebenenfalls mit Phenyl oder Benzyl substituiert ist, wobei jeder der Arylreste unsubstituiert oder mit einem Halogenatom, NO₂, CF₃, einem C₁₋₆-Alkylrest, einem C₁₋₆-Alkoxyrest, CN oder CO₂R⁷ substituiert ist;
R⁶ -Z-COOR⁸ oder -Z-CONR⁷R⁷ ist;
Z eine Einfachbindung, eine Vinylgruppe, -CH₂-O-CH₂-, eine gegebenenfalls mit einem C₁₋₆-Alkylrest, einer oder zwei Benzylgruppen, einer Thienylmethyl- oder einer Furylmethylgruppe substituierte Methylengruppe, -C(O)NHCR⁹-, wobei R⁹ ein Wasserstoffatom ist, ein C₁₋₆-Alkylrest, eine Phenyl-, Benzyl-, Thienylmethyl- oder Furylmethylgruppe ist;
W CₙF₂ₙ₊₁ ist, wobei n 1 bis 3 ist;
A -(CH₂)ₘ-, -CH=CH-, -O(CH₂)ₙ- oder -S(CH₂)ₙ- ist;
jeder Rest R⁷ unabhängig ein Wasserstoffatom, ein C₁₋₆-Alkylrest oder eine (CH₂)ₘ-Phenylgruppe ist, wobei m 0 bis 4 ist; und
R⁸ ein Wasserstoffatom, ein C₁₋₆-Alkylrest, oder 2-Di(C₁₋₆-alkyl)-amino-2-oxoethyl ist;
oder ein pharmazeutisch verträgliches Salz davon.

2. Verfahren nach Anspruch 1, wobei das Diuretikum Hydrochlorthiazid oder Furosemid ist.

3. Verfahren zur Herstellung einer Arzneimittelzusammensetzung umfassend das Zusammenbringen eines Calciumkanalblockers, eines pharmazeutisch verträglichen Trägers und eines Angiotensin-II-Rezeptorantagonisten der Formel (I), wie in Anspruch 1 beschrieben.

4. Verfahren nach Anspruch 3, wobei der Calciumkanalblocker Nifedipin, Diltiazem oder Verapimil ist.

5. Verfahren zur Herstellung einer Arzneimittelzusammensetzung umfassend das Zusammenbringen eines β-Adrenorezeptorblockers, eines pharmazeutisch verträglichen Trägers und eines Angiotensin-II-Rezeptorantagonisten der Formel (I), wie in Anspruch 1 beschrieben.

6. Verfahren nach Anspruch 5, wobei der β-Adrenorezeptorblocker Propanolol ist.

7. Verfahren zur Herstellung einer Arzneimittelzusammensetzung umfassend das Zusammenbringen eines Renininhibitors, eines pharmazeutisch verträglichen Trägers und eines Angiotensin-II-Rezeptorantagonisten der Formel (I), wie in Anspruch 1 beschrieben.

8. Verfahren nach Anspruch 7, wobei der Renininhibitor Enalkinen ist.

9. Verfahren zur Herstellung einer Arzneimittelzusammensetzung umfassend das Zusammenbringen eines Inhibitors des Enzyms, das Angiotensin umsetzt, eines pharmazeutisch verträglichen Trägers und eines Angiotensin-II-Rezeptorantagonisten der Formel (I), wie in Anspruch 1 beschrieben.

10. Verfahren nach Anspruch 9, wobei der Inhibitor des Enzyms, das Angiotensin umsetzt, Captopril oder Enalapril ist.

11. Verfahren nach einem der Ansprüche 1 bis 10, wobei der Angiotensin-II-Rezeptorantagonist (E)-3-[2-n-butyl-1-{(4-carboxyphenyl)methyl}-1H-imidazol-5-yl]-2-(2-thienyl)methyl-2-propensäure ist oder ein pharmazeutisch verträgliches Salz davon.

12. Verfahren nach einem der Ansprüche 1 bis 10, wobei der Angiotensin-II-Rezeptorantagonist (E)-3-[2-n-butyl-1-{(4-carboxyphenyl)methyl}-1H-imidazol-5-yl]-2-(2-thienyl)methyl-2-propensäuremethansulfonat ist.

13. Verfahren nach einem der Ansprüche 1 bis 10, wobei der Angiotensin-II-Rezeptorantagonist (E)-3-[2-n-butyl-1-{(4-carboxynaphth-1-yl)methyl}-1H-imidazol-5-yl]-2-(2-thienyl)methyl-2-propensäure ist oder ein pharmazeutisch verträgliches Salz davon.

14. Verwendung eines Angiotensin-II-Rezeptorantagonisten der Formel (I), wie in Anspruch 1 beschrieben, und eines Diuretikums zur Herstellung eines Arzneimittels zur Behandlung von Bluthochdruck.

15. Verwendung nach Anspruch 14, wobei das Diuretikum Hydrochlorthiazid oder Furosemid ist.

16. Verwendung eines Angiotensin-II-Rezeptorantagonisten der Formel (I), wie in Anspruch 1 beschrieben, und eines Calciumkanalblockers zur Herstellung eines Arzneimittels zur Behandlung von Bluthochdruck.

17. Verwendung nach Anspruch 16, wobei der Calciumkanalblocker Nifedipin, Diltiazem oder Verapimil ist.

18. Verwendung eines Angiotensin-II-Rezeptorantagonisten der Formel (I), wie in Anspruch 1 beschrieben, und eines β-Adrenorezeptorblockers zur Herstellung eines Arzneimittels zur Behandlung von Bluthochdruck.

19. Verwendung nach Anspruch 18, wobei der β-Adrenorezeptorblocker Propanolol ist.

20. Verwendung eines Angiotensin-II-Rezeptorantagonisten der Formel (I), wie in Anspruch 1 beschrieben, und eines Renininhibitors zur Herstellung eines Arzneimittels zur Behandlung von Bluthochdruck.

21. Verwendung nach Anspruch 20, wobei der Renininhibitor Enalkinen ist.

22. Verwendung eines Angiotensin-II-Rezeptorantagonisten der Formel (I), wie in Anspruch 1 beschrieben, und eines Inhibitors des Enzyms, das Angiotensin umsetzt, zur Herstellung eines Arzneimittels zur Behandlung von Bluthochdruck.

23. Verwendung nach Anspruch 22, wobei der Inhibitor des Enzyms, das Angiotensin umsetzt, Captopril oder Enalapril ist.

24. Verwendung nach einem der Ansprüche 14 bis 23, wobei der Angiotensin-II-Rezeptorantagonist (E)-3-[2-n-butyl-1-{(4-carboxyphenyl)methyl}-1H-imidazol-5-yl]-2-(2-thienyl)methyl-2-propensäure ist oder ein pharmazeutisch verträgliches Salz davon.

25. Verwendung nach einem der Ansprüche 14 bis 23, wobei der Angiotensin-II-Rezeptorantagonist (E)-3-[2-n-butyl-1-{(4-carboxyphenyl)methyl}-1H-imidazol-5-yl]-2-(2-thienyl)methyl-2-propensäuremethansulfonat ist.

26. Verwendung nach einem der Ansprüche 14 bis 23, wobei der Angiotensin-II-Rezeptorantagonist (E)-3-[2-n-butyl-1-{(4-carboxynaphth-1-yl)methyl}-1H-imidazol-5-yl]-2-(2-thienyl)-methyl-2-propensäure ist oder ein pharmazeutisch verträgliches Salz davon.

27. Arzneimittelzusammensetzung umfassend ein Diuretikum, einen pharmazeutisch verträglichen Träger und einen Angiotensin-II-Rezeptorantagonisten der Formel (I): 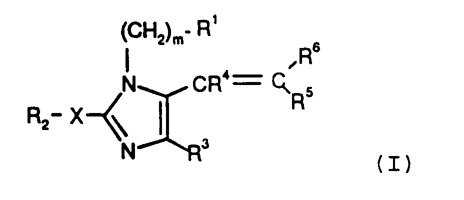 in der
R¹ ein Adamantyl-, Phenyl-, Biphenyl- oder Naphthylrest ist, wobei jeweils der Arylrest unsubstituiert oder mit einem bis drei Substituenten substituiert ist, ausgewählt aus Cl, Br, F, I, einem C₁₋₆-Alkylrest, einer Nitrogruppe, einem Rest A-CO₂R⁷, Tetrazol-5-yl, einem C₁₋₆-Alkoxyrest, einer Hydroxygruppe, einem SC₁₋₆-Alkylrest, SO₂NHR⁷, NHSO₂R⁷, SO₃H, CONR⁷R⁷, CN, einem SO₂C₁₋₆-Alkylrest, PO(OR⁷)₂, NR⁷R⁷, NR⁷COH, einem NR⁷COC₁₋₆-Alkylrest, NR⁷CON(R⁷)₂, NR⁷COW, W oder SO₂W;
m 0 bis 4 ist;
R² ein C₂₋₁₀-Alkylrest, ein C₃₋₁₀-Alkenylrest, ein C₃₋₁₀-Alkynylrest, ein C₃₋₆-Cycloalkylrest oder ein (CH₂)₀₋₈-Phenylrest ist, der unsubstituiert oder mit einem bis drei Substituenten substituiert ist, ausgewählt aus einem C₁₋₆-Alkylrest, einer Nitrogruppe, Cl, Br, F, I, einer Hydroxygruppe, einem C₁₋₆-Alkoxyrest, NR⁷R⁷, CO₂R⁷, CN, CONR⁷R⁷, W, Tetrazol-5-yl, einem NR⁷COC₁₋₆-Alkylrest, NR⁷COW, einem SC₁₋₆-Alkylrest, SO₂W oder einem SO₂C₁₋₆-Alkylrest;
X eine Einfachbindung, S, NR⁷ oder O ist;
R³ ein Wasserstoffatom, Cl, Br, F, I, CHO, eine Hydroxymethylgruppe, COOR⁷, CONR⁷R⁷, NO₂, W, CN, NR⁷R⁷ oder ein Phenylrest ist;
R⁴ und R⁵ unabhängig voneinander ein Wasserstoffatom, ein C₁₋₆-Alkylrest, eine Thienyl-Y-, Furyl-Y-, Pyrazolyl-Y-, Imidazolyl-Y-, Pyrrolyl-Y-, Triazolyl-Y-, Oxazolyl-Y-, Isoxazolyl-Y-, Thiazolyl-Y-, Pyridyl-Y- oder Tetrazolyl-Y-Gruppe ist, mit der Ausnahme, daß R⁴ und R⁵ nicht beide ausgewählt sind aus einem Wasserstoffatom und einem C₁₋₆-Alkylrest und jeder heterocyclische Ring unsubstituiert oder mit einem C₁₋₆-Alkylrest, einem C₁₋₆-Alkoxyrest, Cl, Br, F, I, NR⁷R⁷, CO₂R⁷, SO₂NHR⁷, SO₃H, CONR²R⁷, OH, NO₂, W, SO₂W, einem SC₁₋₆-Alkylrest, einem SO₂C₁₋₆-Alkylrest, NR⁷COH, NR⁷COW oder einem NR⁷COC₁₋₆-Alkylrest substituiert ist;
Y eine Einfachbindung, O, S oder ein unverzweigter oder verzweigter C₁₋₆-Alkylrest ist, der gegebenenfalls mit Phenyl oder Benzyl substituiert ist, wobei jeder der Arylreste unsubstituiert oder mit einem Halogenatom, NO₂, CF₃, einem C₁₋₆-Alkylrest, einem C₁₋₆-Alkoxyrest, CN oder CO₂R⁷ substituiert ist;
R⁶ -Z-COOR⁸ oder -Z-CONR⁷R⁷ ist;
Z eine Einfachbindung, eine Vinylgruppe, -CH₂-O-CH₂-, eine gegebenenfalls mit einem C₁₋₆-Alkylrest, einer oder zwei Benzylgruppen, einer Thienylmethyl- oder einer Furylmethylgruppe substituierte Methylengruppe, -C(O)NHCR⁹-, wobei R⁹ ein Wasserstoffatom ist, ein C₁₋₆-Alkylrest, eine Phenyl-, Benzyl-, Thienylmethyl- oder Furylmethylgruppe ist;
W CₙF₂ₙ₊₁ ist, wobei n 1 bis 3 ist;
A -(CH₂)ₘ-, -CH=CH-, -O(CH₂)ₙ- oder -S(CH₂)ₙ- ist;
jeder Rest R⁷ unabhängig ein Wasserstoffatom, ein C₁₋₆-Alkylrest oder eine (CH₂)ₘ-Phenylgruppe ist, wobei m 0 bis 4 ist; und
R⁸ ein Wasserstoffatom, ein C₁₋₆-Alkylrest, oder 2-Di(C₁₋₆-alkyl)-amino-2-oxoethyl ist; oder ein pharmazeutisch verträgliches Salz davon.

28. Arzneimittelzusammensetzung nach Anspruch 27, wobei das Diuretikum Hydrochlorthiazid oder Furosemid ist.

29. Arzneimittelzusammensetzung umfassend einen Calciumkanalblocker, einen pharmazeutisch verträglichen Träger und einen Angiotensin-II-Rezeptorantagonisten der Formel (I), wie in Anspruch 27 beschrieben.

30. Arzneimittelzusammensetzung nach Anspruch 29, wobei der Calciumkanalblocker Nifedipin, Diltiazem oder Verapimil ist.

31. Arzneimittelzusammensetzung umfassend einen β-Adrenorezeptorblocker, einen pharmazeutisch verträglichen Träger und einen Angiotensin-II-Rezeptorantagonisten der Formel (I), wie in Anspruch 27 beschrieben.

32. Arzneimittelzusammensetzung nach Anspruch 31, wobei der β-Adrenorezeptorblocker Propanolol ist.

33. Arzneimittelzusammensetzung umfassend einen Renininhibitor, einen pharmazeutisch verträglichen Träger und einen Angiotensin-II-Rezeptorantagonisten der Formel (I), wie in Anspruch 27 beschrieben.

34. Arzneimittelzusammensetzung nach Anspruch 33, wobei der Renininhibitor Enalkinen ist.

35. Arzneimittelzusammensetzung umfassend einen Inhibitor des Enzyms, das Angiotensin umsetzt, einen pharmazeutisch verträglichen Träger und einen Angiotensin-II-Rezeptorantagonisten der Formel (I) wie in Anspruch 27 beschrieben.

36. Arzneimittelzusammensetzung nach Anspruch 35, wobei der Inhibitor des Enzyms, das Angiotensin umsetzt, Captopril oder Enalapril ist.

37. Arzneimittelzusammensetzung nach einem der Ansprüche 27 bis 36, wobei der Angiotensin-II-Rezeptorantagonist (E)-3-[2-n-butyl-1-{(4-carboxyphenyl)methyl}-1H-imidazol-5-yl]-2- (2-thienyl)-methyl-2-propensäure ist oder ein pharmazeutisch verträgliches Salz davon.

38. Arzneimittelzusammensetzung nach einem der Ansprüche 27 bis 36, wobei der Angiotensin-II-Rezeptorantagonist (E)-3-[2-n-butyl-1-{(4-carboxyphenyl)methyl}-1H-imidazol-5-yl]-2-(2-thienyl)-methyl-2-propensäuremethansulfonat ist.

39. Arzneimittelzusammensetzung nach einem der Ansprüche 27 bis 36, wobei der Angiotensin-II-Rezeptorantagonist (E)-3-[2-n-butyl-1-{(4-carboxynaphth-1-yl)methyl}-1H-imidazol-5-yl]-2-(2-thienyl)-methyl-2-propensäure ist oder ein pharmazeutisch verträgliches Salz davon.

## Revendications (Revendications pour l'(les) Etat(s) contractant(s) suivant(s): AT, BE, CH, DE, DK, FR, GB, IT, LI, LU, MC, NL, SE)

1. Composition pharmaceutique comprenant un agent diurétique, un support pharmaceutiquement acceptable et un antagoniste des récepteurs d'angiotensine II de formule (I) : 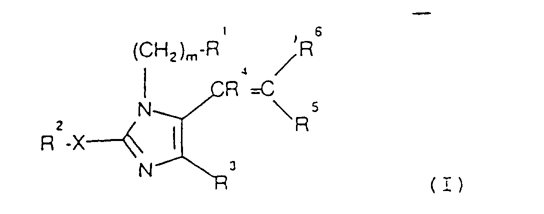 dans laquelle :
R¹ représente un groupe adamantyle, phényle, biphényle ou naphtyle, chaque groupe aryle étant non substitué ou substitué avec un à trois substituants choisis entre Cl, Br, F, I, des substituants alkyle en C₁ à C₆, nitro, A-CO₂R⁷, tétrazole-5-yle, alkoxy en C₁ à C₆, hydroxy, S-alkyle en C₁ à C₆, SO₂NHR⁷, NHSO₂R⁷, SO₃H, CONR⁷R⁷, CN, SO₂-(alkyle en C₁ à C₆), PO(OR⁷)₂, NR⁷R⁷, -NR⁷COH, NR⁷CO-(alkyle en C₁ à C₆), NR⁷CON(R⁷)₂, NR⁷COW, W et SO₂W ;
m a une valeur de 0 à 4 ;
R² représente un groupe alkyle en C₂ à C₁₀, alcényle en C₃ à C₁₀, alcynyle en C₃ à C₁₀, cycloalkyle en C₃ à C₆ ou (CH₂)₀₋₈phényle non substitué ou substitué avec un à trois substituants choisis entre des substituants alkyle en C₁ à C₆, nitro, Cl, Br, F, I, hydroxy, alkoxy en C₁ à C₆, NR⁷R⁷, CO₂R⁷, CN, CONR⁷R⁷, W, tétrazole-5-yle, NR⁷CO-(alkyle en C₁ à C₆), NR⁷COW, S-(alkyle en C₁ à C₆), SO₂W et SO₂-(alkyle en C₁ à C₆) ;
X représente une liaison simple, S, un groupe NR⁷ ou O ;
R³ représente l'hydrogène, Cl, Br, F, I, un groupe CHO, hydroxyméthyle, COOR⁷, CONR⁷R⁷, NO₂, W, CN, NR⁷R⁷ ou phényle ;
R⁴ et R⁵ représentent indépendamment l'hydrogène, un groupe alkyle en C₁ à C₆, thiényl-Y-, furyl-Y-, pyrazolyl-Y-, imidazolyl-Y-, pyrrolyl-Y-, triazolyl-Y-, oxazolyl-Y-, isoxazolyl-Y-, thiazolyl-Y-, pyridyl-Y-, ou tétrazolyl-Y-, sauf que R⁴ et R⁵ ne sont pas choisis l'un et l'autre entre l'hydrogène et un groupe alkyle en C₁ à C₆ et chaque noyau hétérocyclique est non substitué ou substitué avec un substituant alkyle en C₁ à C₆, alkoxy en C₁ à C₆, Br, F, I, NR⁷R⁷,CO₂R⁷, SO₂NHR⁷, SO₃H, CONR²R⁷, OH, NO₂, W, SO₂W, S-(alkyle en C₁ à C₆), SO₂-(alkyle en C₁ à C₆), NR⁷COH, NR⁷COW ou NR⁷CO-(alkyle en C₁ à C₆) ;
Y représente une liaison simple, O, S, ou un groupe alkyle en C₁ à C₆ qui est droit ou ramifié facultativement substitué avec un substituant phényle ou benzyle, chacun des groupes aryle étant non substitué ou substitué avec un substituant halogéno, NO₂, CF₃, alkyle en C₁ à C₆, alkoxy en C₁ à C₆, CN ou CO₂R⁷ ;
R⁶ représente un groupe -Z-COOR⁸ ou Z-CONR⁷R⁷ ;
Z représente une liaison simple, un groupe vinyle, -CH₂-O-CH₂-, méthylène facultativement substitué avec le substituant alkyle en C₁ à C₆, un ou deux groupes benzyle, thiénylméthyle ou furylméthyle, ou un groupe -C(O)NHCR⁹-, dans lequel R⁹ représente H, un groupe alkyle en C₁ à C₆, phényle, benzyle, thiénylméthyle ou furylméthyle ;
W représente un groupe CₙF₂ₙ₊₁, dans lequel n a une valeur de 1 à 3 ;
A représente un groupe -(CH₂)ₘ- -CH=CH-, -O(CH₂)ₙ-, ou -S(CH₂)ₙ- ;
chaque groupe R⁷ représente indépendamment l'hydrogène, un groupe alkyle en C₁ à C₆ ou (CH₂)ₘphényle dans lequel m a une valeur de 0 à 4 ; et
R⁸ représente l'hydrogène, un groupe alkyle en C₁ à C₆ ou 2-di(alkyle en C₁ à C₆)amino-2-oxoéthyle ;
ou un de ses sels pharmaceutiquement acceptables.

2. Composition pharmaceutique suivant la revendication 1, dans laquelle l'agent diurétique est l'hydrochlorothiazide ou le furosémide.

3. Composition pharmaceutique comprenant un agent de blocage du canal calcium, un support pharmaceutiquement acceptable et un antagoniste des récepteurs d'angiotensine II de formule (I), répondant à la définition suivant la revendication 1.

4. Composition pharmaceutique suivant la revendication 3, dans laquelle l'agent de blocage du canal calcium est la nifédipine, le diltiazem, ou le vérapamil.

5. Composition pharmaceutique comprenant un agent de blocage des β-adrénorécepteurs, un support pharmaceutiquement acceptable et un antagoniste des récepteurs d'angiotensine II de formule (I), répondant à la définition suivant la revendication 1.

6. Composition pharmaceutique suivant la revendication 5, dans laquelle l'agent de blocage des β-adrénocepteurs est le propranolol.

7. Composition pharmaceutique comprenant un inhibiteur de rénine, un support pharmaceutiquement acceptable et un antagoniste des récepteurs d'angiotensine II de formule (I), répondant à la définition suivant la revendication 1.

8. Composition pharmaceutique suivant la revendication 7, dans laquelle l'inhibiteur de rénine est l'énalkinène.

9. Composition pharmaceutique comprenant un inhibiteur de l'enzyme de conversion d'angiotensine, un support pharmaceutiquement acceptable et un antagoniste des récepteurs d'angiotensine II de formule (I), répondant à la définition suivant la revendication 1.

10. Composition pharmaceutique suivant la revendication 9, dans laquelle l'inhibiteur de conversion d'angiotensine est le captopril ou l'énalapril.

11. Composition pharmaceutique suivant l'une quelconque des revendications 1 à 10, dans laquelle l'antagoniste du récepteur d'angiotensine II est l'acide (E)-3-[2-n-butyl-1-{(4-carboxyphényl)méthyl}-1H-imidazole-5-yl]-2-(2-thiényl)-méthyl-2-propénoïque ou un de ses sels pharmaceutiquement acceptables.

12. Composition pharmaceutique suivant l'une quelconque des revendications 1 à 10, dans laquelle l'antagoniste des récepteurs d'angiotensine II est le méthanesulfonate d'acide (E)-3-[2-n-butyl-1-{(4-carboxyphényl)méthyl}-1H-imidazole-5-yl]-2-(2-thiényl)-méthyl-2-propénoïque.

13. Composition pharmaceutique suivant l'une quelconque des revendications 1 à 10, dans laquelle l'antagoniste des récepteurs d'angiotensine II est l'acide (E)-3-[2-n-butyl-1-{(4-carboxynapht-1-yl)méthyl}-1H-imidazole-5-yl]-2-(2-thiényl)-méthyl-2-propénoïque ou un de ses sels pharmaceutiquement acceptables.

14. Utilisation d'un antagoniste des récepteurs d'angiotensine II de formule (I), répondant à la définition suivant la revendication 1, et d'un agent diurétique dans la production d'un médicament pour le traitement de l'hypertension.

15. Utilisation suivant la revendication 14, dans laquelle l'agent diurétique est l'hydrochlorothiazide ou le furosémide.

16. Utilisation d'un antagoniste des récepteurs d'angiotensine II de formule (I), répondant à la définition suivant la revendication 1, et d'un agent de blocage du canal calcium dans la production d'un médicament destiné au traitement de l'hypertension.

17. Utilisation suivant la revendication 16, dans laquelle l'agent de blocage du canal calcium est la nifédipine, le diltiazem ou le vérapamil.

18. Utilisation d'un antagoniste des récepteurs d'angiotensine II de formule (I), répondant à la définition suivant la revendication 1, et d'un agent de blocage des β-adrénocepteurs dans la production d'un médicament destiné au traitement de l'hypertension.

19. Utilisation suivant la revendication 18, dans laquelle l'agent de blocage des β-adrénorécepteurs est le propranolol.

20. Utilisation d'un antagoniste des récepteurs d'angiotensine II de formule (I), répondant à la définition suivant la revendication 1, et d'un inhibiteur de rénine dans la production d'un médicament destiné au traitement de l'hypertension.

21. Utilisation suivant la revendication 20, dans laquelle l'inhibiteur de rénine est l'énalkinène.

22. Utilisation d'un antagoniste des récepteurs d'angiotensine II de formule (I) répondant à la définition suivant la revendication 1, et d'un inibiteur d'enzyme de conversion de l'angiotensine dans la production d'un médicament destiné au traitement de l'hypertension.

23. Utilisation suivant la revendication 22, dans laquelle l'inhibiteur d'enzyme de conversion de l'angiotensine est le captopril ou l'énalapril.

24. Utilisation suivant l'une quelconque des revendications 14 à 23, dans laquelle l'antagoniste des récepteurs d'angiotensine II est l'acide (E)-3-[2-n-butyl-1-{(4-carboxyphényl)méthyl}-1H-imidazole-5-yl]-2-(2-thiényl)méthyl-2-propénoïque ou un de ses sels pharmaceutiquement acceptables.

25. Utilisation suivant l'une quelconque des revendications 14 à 23, dans laquelle l'antagoniste des récepteurs d'angiotensine II est le méthanesulfonate d'acide (E)-3-[2-n-butyl-1-{(4-carboxyphényl)méthyl}-1H-imidazole-5-yl]-2-(2-thiényl)-méthyl-2-propénoïque.

26. Utilisation suivant l'une quelconque des revendications 14 à 23, dans laquelle l'antagoniste des récepteurs d'angiotensine II est l'acide (E)-3-[2-n-butyl-1-{(4-carboxynapht-1-yl)méthyl}-1H-imidazole-5-yl]-2-(2- thiényl)-méthyl-2-propénoïque ou un de ses sels pharmaceutiquement acceptables.

## Revendications (Revendications pour l'(les) Etat(s) contractant(s) suivant(s): ES, GR)

1. Procédé pour la préparation d'une composition pharmaceutique, qui comprend la mise en association d'un agent diurétique, d'un support pharmaceutiquement acceptable et d'un antagoniste des récepteurs d'angiotensine II de formule (I) : 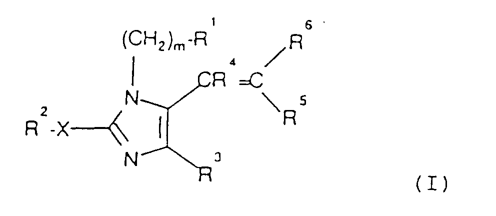 dans laquelle :
R¹ représente un groupe adamantyle, phényle, biphényle ou naphtyle, chaque groupe aryle étant non substitué ou substitué avec un à trois substituants choisis entre Cl, Br, F, I, des substituants alkyle en C₁ à C₆, nitro, A-CO₂R⁷, tétrazole-5-yle, alkoxy en C₁ à C₆, hydroxy, S-alkyle en C₁ à C₆, SO₂NHR⁷, NHSO₂R⁷, SO₃H, CONR⁷R⁷, CN, SO₂-(alkyle en C₁ à C₆), PO(OR⁷)₂, NR⁷R⁷, -NR⁷COH, NR⁷CO-(alkyle en C₁ à C₆), NR⁷CON(R⁷)₂, NR⁷COW, W et SO₂W ;
m a une valeur de 0 à 4 ;
R² représente un groupe alkyle en C₂ à C₁₀, alcényle en C₃ à C₁₀, alcynyle en C₃ à C₁₀, cycloalkyle en C₃ à C₆ ou (CH₂)₀₋₈phényle non substitué ou substitué avec un à trois substituants choisis entre des substituants alkyle en C₁ à C₆, nitro, Cl, Br, F, I, hydroxy, alkoxy en C₁ à C₆, NR⁷R⁷, CO₂R⁷, CN, CONR⁷R⁷, W, tétrazole-5-yle, NR⁷CO-(alkyle en C₁ à C₆), NR⁷COW, S-(alkyle en C₁ à C₆), SO₂W et SO₂-(alkyle en C₁ à C₆) ;
X représente une liaison simple, S, un groupe NR⁷ ou O ;
R³ représente l'hydrogène, Cl, Br, F, I, un groupe CHO, hydroxyméthyle, COOR⁷, CONR⁷R⁷, NO₂, W, CN, NR⁷R⁷ ou phényle ;
R⁴ et R⁵ représentent indépendamment l'hydrogène, un groupe alkyle en C₁ à C₆, thiényl-Y-, furyl-Y-, pyrazolyl-Y-, imidazolyl-Y-, pyrrolyl-Y-, triazolyl-Y-, oxazolyl-Y-, isoxazolyl-Y-, thiazolyl-Y-, pyridyl-Y-, ou tétrazolyl-Y-, sauf que R⁴ et R⁵ ne sont pas choisis l'un et l'autre entre l'hydrogène et un groupe alkyle en C₁ à C₆, et chaque noyau hétérocyclique est non substitué ou substitué avec un substituant alkyle en C₁ à C₆, alkoxy en C₁ à C₆, Cl, Br, F, I, NR⁷R⁷,CO₂R⁷, SO₂NHR⁷, SO₃H, CONR²R⁷, OH, NO₂, W, SO₂W, S-(alkyle en C₁ à C₆), SO₂-(alkyle en C₁ à C₆), NR⁷COH, NR⁷COW ou NR⁷CO-(alkyle en C₁ à C₆) ;
Y représente une liaison simple, O, S, ou un groupe alkyle en C₁ à C₆ qui est droit ou ramifié ou facultativement substitué avec un substituant phényle ou benzyle, chacun des groupes aryle étant non substitué ou substitué avec un substituant halogéno, NO₂, CF₃, alkyle en C₁ à C₆, alkoxy en C₁ à C₆, CN ou CO₂R⁷ ;
R⁶ représente un groupe -Z-COOR⁸ ou Z-CONR⁷R⁷ ;
Z représente une liaison simple, un groupe vinyle, -CH₂-O-CH₂-, méthylène facultativement substitué avec un substituant alkyle en C₁ à C₆, un ou deux groupes benzyle, thiénylméthyle ou furylméthyle, ou -C(O)NHCR⁹-, dans lequel R⁹ représente H, un groupe alkyle en C₁ à C₆, phényle, benzyle, thiénylméthyle ou furylméthyle ;
W représente un groupe CₙF₂ₙ₊₁, dans lequel n a une valeur de 1 à 3 ;
A représente un groupe -(CH₂)ₘ- -CH=CH-, -O(CH₂)ₙ- ou -S(CH₂)ₙ- ;
chaque groupe R⁷ représente indépendamment l'hydrogène, un groupe alkyle en C₁ à C₆ ou (CH₂)ₘphényle dans lequel m a une valeur de 0 à 4 ; et
R⁸ représente l'hydrogène, un groupe alkyle en C₁ à C₆ ou 2-di(alkyle en C₁ à C₆)amino-2-oxoéthyle ;
ou un de ses sels pharmaceutiquement acceptables.

2. Procédé suivant la revendication 1, dans lequel le diurétique est l'hydrochlorothiazide ou le furosémide.

3. Procédé pour la préparation d'une composition pharmaceutique, qui comprend la mise en association d'un agent de blocage du canal calcium, d'un support pharmaceutiquement acceptable et d'un antagoniste des récepteurs d'angiotensine II de formule (I), répondant à la définition suivant la revendication 1.

4. Procédé suivant la revendication 3, dans lequel l'agent de blocage du canal calcium est la nifédipine, le diltiazem, ou le vérapamil.

5. Procédé pour la préparation d'une composition pharmaceutique, qui comprend la mise en association d'un agent de blocage des β-adrénorécepteurs, d'un support pharmaceutiquement acceptable et d'un antagoniste des récepteurs d'angiotensine II de formule (I), répondant à la définition suivant la revendication 1.

6. Procédé suivant la revendication 5, dans laquelle l'agent de blocage des β-adrénocepteurs est le propranolol.

7. Procédé pour la préparation d'une composition pharmaceutique, qui comprend la mise en association d'un inhibiteur de rénine, d'un support pharmaceutiquement acceptable et d'un antagoniste des récepteurs d'angiotensine II de formule (I), répondant à la définition suivant la revendication 1.

8. Procédé suivant la revendication 7, dans lequel l'inhibiteur de rénine est l'énalkinène.

9. Procédé pour la préparation d'une composition pharmaceutique, qui comprend la mise en association d'un inhibiteur de l'enzyme de conversion d'angiotensine, d'un support pharmaceutiquement acceptable et d'un antagoniste des récepteurs d'angiotensine II de formule (I), répondant à la définition suivant la revendication 1.

10. Procédé suivant la revendication 9, dans lequel l'inhibiteur de conversion d'angiotensine est le captopril ou l'énalapril.

11. Procédé suivant l'une quelconque des revendications 1 à 10, dans lequel l'antagoniste du récepteur d'angiotensine II est l'acide (E)-3-[2-n-butyl-1-{(4-carboxyphényl)méthyl}-1H-imidazole-5-yl]-2-(2-thiényl)-méthyl-2-propénoïque ou un de ses sels pharmaceutiquement acceptables.

12. Procédé suivant l'une quelconque des revendications 1 à 10, dans lequel l'antagoniste des récepteurs d'angiotensine II est le méthanesulfonate d'acide (E)-3-[2-n-butyl-1-{(4-carboxyphényl)méthyl}-1H-imidazole-5-yl]-2-(2-thiényl)-méthyl-2-propénoïque.

13. Procédé suivant l'une quelconque des revendications 1 à 10, dans lequel l'antagoniste des récepteurs d'angiotensine II est l'acide (E)-3-[2-n-butyl-1-{(4-carboxynapht-1-yl)méthyl}-1H-imidazole-5-yl]-2-(2-thiényl)-méthyl-2-propénoïque ou un de ses sels pharmaceutiquement acceptables.

14. Utilisation d'un antagoniste des récepteurs d'angiotensine II de formule (I), répondant à la définition suivant la revendication 1, et d'un agent diurétique dans la production d'un médicament destiné au traitement de l'hypertension.

15. Utilisation suivant la revendication 14, dans laquelle l'agent diurétique est l'hydrochlorothiazide ou le furosémide.

16. Utilisation d'un antagoniste des récepteurs d'angiotensine II de formule (I), répondant à la définition suivant la revendication 1, et d'un agent de blocage du canal calcium dans la production d'un médicament destiné au traitement de l'hypertension.

17. Utilisation suivant la revendication 16, dans laquelle l'agent de blocage du canal calcium est la nifédipine, le diltiazem ou le vérapamil.

18. Utilisation d'un antagoniste des récepteurs d'angiotensine II de formule (I), répondant à la définition suivant la revendication 1, et d'un agent de blocage des β-adrénocepteurs dans la production d'un médicament destiné au traitement de l'hypertension.

19. Utilisation suivant la revendication 18, dans laquelle l'agent de blocage des β-adrénorécepteurs est le propranolol.

20. Utilisation d'un antagoniste des récepteurs d'angiotensine II de formule (I), répondant à la définition suivant la revendication 1, et d'un inhibiteur de rénine dans la production d'un médicament destiné au traitement de l'hypertension.

21. Utilisation suivant la revendication 20, dans laquelle l'inhibiteur de rénine est l'énalkinène.

22. Utilisation d'un antagoniste des récepteurs d'angiotensine II de formule (I) répondant à la définition suivant la revendication 1, et d'un inhibiteur d'enzyme de conversion de l'angiotensine dans la production d'un médicament destiné au traitement de l'hypertension.

23. Utilisation suivant la revendication 22, dans laquelle l'inhibiteur d'enzyme de conversion de l'angiotensine est le captopril ou l'énalapril.

24. Utilisation suivant l'une quelconque des revendications 14 à 23, dans laquelle l'antagoniste des récepteurs d'angiotensine II est l'acide (E)-3-[2-n-butyl-1-{(4-carboxyphényl)méthyl}-1H-imidazole-5-yl]-2-(2-thiényl)méthyl-2-propénoïque ou un de ses sels pharmaceutiquement acceptables.

25. Utilisation suivant l'une quelconque des revendications 14 à 23, dans laquelle l'antagoniste des récepteurs d'angiotensine II est le méthanesulfonate d'acide (E)-3-[2-n-butyl-1-{(4-carboxyphényl)méthyl}-1H-imidazole-5-yl]-2-(2-thiényl)-méthyl-2-propénoïque.

26. Utilisation suivant l'une quelconque des revendications 14 à 23, dans laquelle l'antagoniste des récepteurs d'angiotensine II est l'acide (E)-3-[2-n-butyl-1-{(4-carboxynapht-1-yl)méthyl}-1H-imidazole-5-yl]-2-(2-thiényl)-méthyl-2-propénoïque ou un de ses sels pharmaceutiquement acceptables.

27. Composition pharmaceutique comprenant un agent diurétique, un support pharmaceutiquement acceptable et un antagoniste des récepteurs d'angiotensine II de formule (I) : 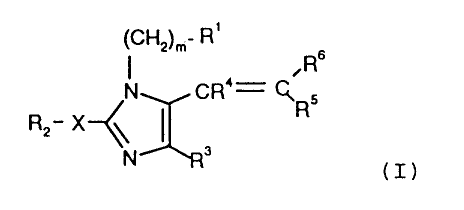 dans laquelle :
R¹ représente un groupe adamantyle, phényle, biphényle ou naphtyle, chaque groupe aryle étant non substitué ou substitué avec un à trois substituants choisis entre les substituants Cl, Br, F, I, alkyle en C₁ à C₆, nitro, A-CO₂R⁷, tétrazole-5-yle, alkoxy en C₁ à C₆, hydroxy, S-alkyle en C₁ à C₆,NHSO₂R⁷, SO₂NHR⁷, NHSO₂R⁷, SO₃H, CONR⁷R⁷, CN, SO₂-(alkyle en C₁ à C₆), NHSO₂R⁷, PO(OR⁷)₂, NR⁷R⁷, NR⁷COH, NR⁷CO-(alkyle en C₁ à C₆), NR⁷CON(R⁷)₂, NR⁷COW, W et SO₂W ;
m a une valeur de 0 à 4 ;
R² représente un groupe alkyle en C₂ à C₁₀, alcényle en C₃ à C₁₀, alcynyle en C₃ à C₁₀, cycloalkyle en C₁ à C₆ ou (CH₂)₀₋₈phényle non substitué ou substitué avec un à trois substituants choisis entre des substituants alkyle en C₁ à C₆, nitro, Cl, Br, F, I, hydroxy, alkoxy en C₁ à C₆, NR⁷R⁷, CO₂R⁷, CN, CONR⁷R⁷, W, tétrazole-5-yle, NR⁷CO-(alkyle en C₁ à C₆), NR⁷COW, S-(alkyle en C₁ à C₆), SO₂W et SO₂-(alkyle en C₁ à C₆) ;
X représente une liaison simple, S, un groupe NR⁷ ou O ;
R³ représente l'hydrogène, Cl, Br, F, I, un groupe CHO, hydroxyméthyle, COOR⁷, CONR⁷R⁷, NO₂, W, CN, NR⁷R⁷ ou phényle ;
R⁴ et R⁵ représentent indépendamment l'hydrogène, un groupe alkyle en C₁ à C₆, thiényl-Y-, furyl-Y-, pyrazolyl-Y-, imidazolyl-Y-, pyrrolyl-Y-, triazolyl-Y-, oxazolyl-Y-, isoxazolyl-Y-, thiazolyl-Y-, pyridyl-Y-, ou tétrazolyl-Y-, sauf que R⁴ et R⁵ ne sont pas choisis l'un et l'autre entre l'hydrogène et un groupe alkyle en C₁ à C₆ et chaque noyau hétérocyclique est non substitué ou substitué avec un substituant alkyle en C₁ à C₆, alkoxy en C₁ à C₆, Cl, Br, F, I, NR⁷R⁷,CO₂R⁷, SO₂NHR⁷, SO₃H, CONR²R⁷, OH, NO₂, W, SO₂W, S-(alkyle en C₁ à C₆), SO₂-(alkyle en C₁ à C₆), NR⁷COH, NR⁷COW ou NR⁷CO-(alkyle en C₁ à C₆) ;
Y représente une liaison simple, O, S, ou un groupe alkyle en C₁ à C₆ qui est droit ou ramifié facultativement sustitué avec un substituant phényle ou benzyle, chacun des groupes aryle étant non substitué ou substitué avec un substituant halogéno, NO₂, CF₃, alkyle en C₁ à C₆, alkoxy en C₁ à C₆, CN ou CO₂R⁷ ;
R⁶ représente un groupe -Z-COOR⁸ ou Z-CONR⁷R⁷ ;
Z représente une liaison simple, un groupe vinyle, -CH₂-O-CH₂-, méthylène facultativement avec un substituant alkyle en C₁ à C₆, un ou deux groupes benzyle, thiénylméthyle ou furylméthyle, ou un groupe -C(O)NHCR⁹-, dans lequel R⁹ représente H, un groupe alkyle en C₁ à C₆, phényle, benzyle, thiénylméthyle ou furylméthyle ;
W représente un groupe CₙF₂ₙ₊₁, dans lequel n a une valeur de 1 à 3 ;
A représente un groupe -(CH₂)ₘ- -CH=CH-, -O(CH₂)ₙ- ou -S(CH₂)ₙ-;
chaque groupe R⁷ représente indépendamment l'hydrogène, un groupe alkyle en C₁ à C₆ ou (CH₂)ₘphényle dans lequel m a une valeur de 0 à 4 ; et
R⁸ représente l'hydrogène, un groupe alkyle en C₁ à C₆ ou 2-di(alkyle en C₁ à C₆)amino-2-oxoéthyle ;
ou un de ses sels pharmaceutiquement acceptables.

28. Composition pharmaceutique suivant la revendication 27, dans laquelle l'agent diurétique est l'hydrochlorothiazide ou le furosémide.

29. Composition pharmaceutique comprenant un agent de blocage du canal calcium, un support pharmaceutiquement acceptable et un antagoniste des récepteurs d'angiotensine II de formule (I), répondant à la définition suivant la revendication 27.

30. Composition pharmaceutique suivant la revendication 29, dans laquelle l'agent de blocage du canal calcium est la nifédipine, le diltiazem ou le vérapamil.

31. Composition pharmaceutique comprenant un agent de blocage des β-adrénorécepteurs, un support pharmaceutiquement acceptable et un antagoniste des récepteurs d'angiotensine II de formule (I), répondant à la définition suivant la revendication 27.

32. Composition pharmaceutique suivant la revendication 31, dans laquelle l'agent de blocage des β-adrénorécepteurs est le propranolol.

33. Composition pharmaceutique comprenant un inhibiteur de rénine, un support pharmaceutiquement acceptable et un antagoniste des récepteurs d'angiotensine II de formule (I), répondant à la définition suivant la revendication 27.

34. Composition pharmaceutique suivant la revendication 33, dans laquelle l'inhibiteur de rénine est l'énalkinène.

35. Composition pharmaceutique comprenant un inhibiteur d'un enzyme de conversion d'angiotensine, un support pharmaceutiquement acceptable et un antagoniste des récepteurs d'angiotensine II de formule (I), répondant à la définition suivant la revendication 27.

36. Composition pharmaceutique suivant la revendication 35, dans laquelle l'inhibiteur d'enzyme de conversion d'angiotensine est le captopril ou l'énalapril.

37. Composition pharmaceutique suivant l'une quelconque des revendications 27 à 36, dans laquelle l'antagoniste des récepteurs d'angiotensine II est l'acide (E)-3-[2-n-butyl-1-{(4-carboxyphényl)méthyl}-1H-imidazole-5-yl]-2-(2-thiényl)-méthyl-2-propénoïque ou un de ses sels pharmaceutiquement acceptables.

38. Composition pharmaceutique suivant l'une quelconque des revendications 27 à 36, dans laquelle l'antagoniste des récepteurs d'angiotensine II est le méthanesulfonate d'acide (E)-3-[2-n-butyl-1-{(4-carboxyphényl)méthyl}-1H-imidazole-5-yl]-2-(2-thiényl)-méthyl-2-propénoïque.

39. Composition pharmaceutique suivant l'une quelconque des revendications 27 à 36, dans laquelle l'antagoniste des récepteurs d'angiotensine II est l'acide (E)-3-[2-n-butyl-1-{(4-carboxynapht-1-yl)méthyl}-1H-imidazole-5-yl]-2-(2-thiényl)-méthyl-2-propénoïque ou un de ses sels pharmaceutiquement acceptables.
